# EUROPEAN PATENT SPECIFICATION

(11) **EP 1 035 208 B1**
(45) Date of publication and mention of the grant of the patent: **29.10.2008**
(21) Application number: 98953027.4
(22) Date of filing: 12.11.1998
(51) Int. Cl.: C12N 15/66, C12N 15/79, C12N 5/06, A61K 48/00, A01K 67/027

(54) **VARIANT LoxP SEQUENCES AND APPLICATION OF THE SAME**
LoxP SEQUENZ-VARIANTEN UND DEREN VERWENDUNG
SEQUENCES LoxP DE VARIANT ET LEUR APPLICATION

(30) Priority: 13.11.1997 JP 33128997; 28.09.1998 JP 27315098
(43) Date of publication of application: 13.09.2000
(73) Proprietor: Dainippon Sumitomo Pharma Co., Ltd., Osaka 541-8524 (JP)
(72) Inventor: SAITO, Izumu, Tokyo 151-0053 (JP); TANAKA, Keiji, Yamashina-ku Kyoto-shi Kyoto 607-8035 (JP)
(74) Representative: Cresswell, Thomas Anthony
(86) International application number: PCT/JP1998/005094
(87) International publication number: WO 1999/025851

(56) References cited:
- LEE YOUNG-SAM ET AL: "A novel mutant loxP containing part of long terminal repeat of HIV-1 in spacer region: Presentation of possible target site for antiviral strategy using site-specific recombinase." BIOCHEMICAL AND BIOPHYSICAL RESEARCH COMMUNICATIONS, vol. 253, no. 3, 30 December 1998 (1998-12-30), pages 588-593, XP002183660 ISSN: 0006-291X
- GWANG LEE et al., "Role of Nucleotide Sequences of loxP Spacer Region in Cre-Mediated Recombination", GENE, (1998), Vol. 216, No. 1, p. 55-65, XP002918422
- Y. SAKAI et al., "Highly Enhanced and Specific Expression for Hepatocellular Carcinoma In Vivo Using Recombinant Adenovirus Vector with cre/loxP System", HEPATOLOGY, (1997), Vol. 26, No. 4, Pt. 2, p. 171A, XP002918424
- RONALD H. HOESS et al., "The Role of the loxP Spacer Region in P1 Site-Specific Recombination", NUCLEIC ACIDS RESEARCH, (1986), Vol. 14, No. 5, p. 2287-2300, XP002918423

### SEQUENCE LISTING

<110>Sumitomo Pharmaceuticals Company,Limited
<120>Mutated Type of loxP and Application thereof
<130>E4317-00
<150>JP 9-331289 and JP 10-273150
   <151>1997-11-13 and 1998-9-28
<160>61
<210>1
   <211>34
   <212>DNA
   <213> Bacteriophage P1
<400>1
   ataacttcgt ataatgtatg ctatacgaag ttat 34
<210>2
   <211>34
   <212>DNA
   <213> Artificial Sequence
<220>
<223> Sequence of Mutant loxP
<400>2
   ataacttcgt atagtgtatg ctatacgaag ttat 34
<210>3
   <211>34
   <212>DNA
   <213> Artificial Sequence
<220>
   <223> Sequence of Mutant loxP
<400>3
   ataacttcgt atattgtatg ctatacgaag ttat 34
<210>4
   <211>34
   <212>DNA
   <213> Artificial Sequence
<220>
   <223> Sequence of Mutant loxP
<400>4
   ataacttcgt atactgtatg ctatacgaag ttat 34
<210>5
   <211>34
   <212>DNA
   <213> Artificial Sequence
<220>
   <223> Sequence of Mutant loxP
<400>5
   ataacttcgt ataacgtatg ctatacgaag ttat 34
<210>6
   <211>34
   <212>DNA
   <213> Artificial Sequence
<220>
   <223> Sequence of Mutant loxP
<400>6
   ataacttcgt ataaagtatg ctatacgaag ttat 34
<210>7
   <211>34
   <212>DNA
   <213> Artificial Sequence
<220>
<223> Sequence of Mutant loxP
<400>7
   ataacttcgt ataaggtatg ctatacgaag ttat 34
<210>8
   <211>34
   <212>DNA
   <213> Artificial Sequence
<220>
<223> Sequence of Mutant loxP
<400>8
   ataacttcgt ataatatatg ctatacgaag ttat 34
<210>9
   <211>34
   <212>DNA
   <213> Artificial Sequence
<220>
   <223> Sequence of Mutant loxP
<400>9
   ataacttcgt ataatctatg ctatacgaag ttat 34
<210>10
   <211>34
   <212>DNA
   <213> Artificial Sequence
<220>
<223> Sequence of Mutant loxP
<400>10
   ataacttcgt ataatttatg ctatacgaag ttat 34
<210>11
   <211>34
   <212>DNA
   <213> Artificial Sequence
<220>
<223> Sequence of Mutant loxP
<400>11
   ataacttcgt ataatgcatg ctatacgaag ttat 34
<210>12
   <211>34
   <212>DNA
   <213> Artificial Sequence
<220>
   <223> Sequence of Mutant loxP
<400>12
   ataacttcgt ataatgaatg ctatacgaag ttat 34
<210>13
   <211>34
   <212>DNA
   < 213> Artificial Sequence
<220>
   <223> Sequence of Mutant loxP
<400>13
   ataacttcgt ataatggatg ctatacgaag ttat 34
<210>14
   <211>34
   <212>DNA
   <213> Artificial Sequence
<220>
   <223> Sequence of Mutant loxP
<400>14
   ataacttcgt ataatgtgtg ctatacgaag ttat 34
<210>15
   <211>34
   <212>DNA
   <213> Artificial Sequence
<220>
<223> Sequence of Mutant loxP
<400>15
   ataacttcgt ataatgtttg ctatacgaag ttat 34
<210>16
   <211>34
   <212>DNA
   <213> Artificial Sequence
<220>
   <223> Sequence of Mutant loxP
<400>16
   ataacttcgt ataatgtctg ctatacgaag ttat 34
<210>17
   <211>34
   <212>DNA
   <213> Artificial Sequence
<220>
   <223> Sequence of Mutant loxP
<400>17
   ataacttcgt ataatgtacg ctatacgaag ttat 34
<210>18
   <211>34
   <212>DNA
   <213> Artificial Sequence
<220>
<223> Sequence of Mutant loxP
<400>18
   ataacttcgt ataatgtaag ctatacgaag ttat 34
<210>19
   <211>34
   <212>DNA
   <213> Artificial Sequence
<220>
   <223> Sequence of Mutant loxP
<400>19
   ataacttcgt ataatgtagg ctatacgaag ttat 34
<210>20
   <211>34
   <212>DNA
   <213> Artificial Sequence
<220>
   <223> Sequence of Mutant loxP
<400>20
   ataacttcgt ataatgtata ctatacgaag ttat 34
<210>21
   <211>34
   <212>DNA
   <213> Artificial Sequence
<220>
   <223> Sequence of Mutant loxP
<400>21
   ataacttcgt ataatgtatc ctatacgaag ttat 34
<210>22
   <211>34
   <212>DNA
   <213> Artificial Sequence
<220>
<223> Sequence of Mutant loxP
<400>22
   ataacttcgt ataatgtatt ctatacgaag ttat 34
<210>23
   <211>34
   <212>DNA
   <213> Artificial Sequence
<220>
   <223> Sequence of Mutant loxP
<400>23
   ataacttcgt ataatgtatg ttatacgaag ttat 34
<210>24
   <211>34
   <212>DNA
   <213> Artificial Sequence
<220>
<223> Sequence of Mutant loxP
<400>24
   ataacttcgt ataatgtatg gtatacgaag ttat 34
<210>25
   <211>34
   <212>DNA
   <213> Artificial Sequence
<220>
   <223> Sequence of Mutant loxP
<400>25
   ataacttcgt ataatgtatg atatacgaag ttat 34
<210>26
   <211>34
   <212>DNA
   <213> Artificial Sequence
<220>
   <223> Sequence of Mutant loxP
<400>26
   ataacttcgt ataacgtata ctatacgaag ttat 34
<210>27
   <211>34
   <212>DNA
   <213> Artificial Sequence
<220>
   <223> Sequence of Mutant loxP
<400>27
   ataacttcgt ataacgtatc ctatacgaag ttat 34
<210>28
   <211>34
   <212>DNA
   <213> Artificial Sequence
<220>
   <223> Sequence of Mutant loxP
<400>28
   ataacttcgt ataacgtatt ctatacgaag ttat 34
<210>29
   <211>34
   <212>DNA
   <213> Artificial Sequence
<220>
   <223> Sequence of Mutant loxP
<400>29
   ataacttcgt ataaagtata ctatacgaag ttat 34
<210>30
   <211>34
   <212>DNA
   <213> Artificial Sequence
<220>
   <223> Sequence of Mutant loxP
<400>30
   ataacttcgt ataaagtatc ctatacgaag ttat 34
<210>31
   <211>34
   <212>DNA
   <213> Artificial Sequence
<220>
   <223> Sequence of Mutant loxP
<400>31
   ataacttcgt ataaagtatt ctatacgaag ttat 34
<210>32
   <211>34
   <212>DNA
   <213> Artificial Sequence
<220>
   <223> Sequence of Mutant loxP
<400>32
   ataacttcgt ataaggtata ctatacgaag ttat 34
<210>33
   <211>34
   <212>DNA
   <213> Artificial Sequence
<220>
   <223> Sequence of Mutant loxP
<400>33
   ataacttcgt ataaggtatc ctatacgaag ttat 34
<210>34
   <211>34
   <212>DNA
   <213> Artificial Sequence
<220>
   <223> Sequence of Mutant loxP
<400>34
   ataacttcgt ataaggtatt ctatacgaag ttat 34
<210>35
   <211>34
   <212>DNA
   <213> Artificial Sequence
<220>
   <223> Sequence of Mutant loxP
<400>35
   ataacttcgt ataatatata ctatacgaag ttat 34
<210>36
   <211>34
   <212>DNA
   <213> Artificial Sequence
<220>
   <223> Sequence of Mutant loxP
<400>36
   ataacttcgt ataatatatc ctatacgaag ttat 34
<210>37
   <211>34
   <212>DNA
   <213> Artificial Sequence
<220>
   <223> Sequence of Mutant loxP
<400>37
   ataacttcgt ataatctata ctatacgaag ttat 34
<210>38
   <211>34
   <212>DNA
   <213> Artificial Sequence
<220>
   <223> Sequence of Mutant loxP
<400>38
   ataacttcgt ataatctatc ctatacgaag ttat 34
<210>39
   <211>34
   <212>DNA
   <213> Artificial Sequence
<220>
   <223> Sequence of Mutant loxP
<400>39
   ataacttcgt ataatttata ctatacgaag ttat 34
<210>40
   <211>34
   <212>DNA
   <213> Artificial Sequence
<220>
   <223> Sequence of Mutant loxP
<400>40
   ataacttcgt ataatttatc ctatacgaag ttat 34
<210>41
   <211>34
   <212>DNA
   <213> Artificial Sequence
<220>
   <223> Sequence of Mutant loxP
<400>41
   ataacttcgt ataatttatt ctatacgaag ttat 34
<210>42
   <211>34
   <212>DNA
   <213> Artificial Sequence
<220>
   <223> Sequence of Mutant loxP
<400>42
   ataacttcgt ataatgcata ctatacgaag ttat 34
<210>43
   <211>34
   <212>DNA
   <213> Artificial Sequence
<220>
   <223> Sequence of Mutant loxP
<400>43
   ataacttcgt ataatgcatc ctatacgaag ttat 34
<210>44
   <211>34
   <212>DNA
   <213> Artificial Sequence
<220>
   <223> Sequence of Mutant loxP
<400>44
   ataacttcgt ataatgaata ctatacgaag ttat 34
<210>45
   <211>34
   <212>DNA
   <213> Artificial Sequence
<220>
   <223> Sequence of Mutant loxP
<400>45
   ataacttcgt ataatgaatc ctatacgaag ttat 34
<210>46
   <211>34
   <212>DNA
   <213> Artificial Sequence
<220>
   <223> Sequence of Mutant loxP
<400>46
   ataacttcgt ataatggata ctatacgaag ttat 34
<210>47
   <211>34
   <212>DNA
   <213> Artificial Sequence
<220>
   <223> Sequence of Mutant loxP
<400>47
   ataacttcgt ataatggatc ctatacgaag ttat 34
<210>48
   <211>34
   <212>DNA
   <213> Artificial Sequence
<220>
   <223> Sequence of Mutant loxP
<400>48
   ataacttcgt ataatggatt ctatacgaag ttat 34
<210>49
   <211>34
   <212>DNA
   <213> Artificial Sequence
<220>
   <223> Sequence of Mutant loxP
<400>49
   ataacttcgt ataatgtgta ctatacgaag ttat 34
<210>50
   <211>34
   <212>DNA
   <213> Artificial Sequence
<220>
   <223> Sequence of Mutant loxP
<400>50
   ataacttcgt ataatgtttc ctatacgaag ttat 34
<210>51
   <211>34
   <212>DNA
   <213> Artificial Sequence
<220>
   <223> Sequence of Mutant loxP
<400>51
   ataacttcgt ataatgtctt ctatacgaag ttat 34
<210>52
   <211>34
   <212>DNA
   <213> Artificial Sequence
<220>
   <223> Sequence of Mutant loxP
<400>52
   ataacttcgt ataatgtaca ctatacgaag ttat 34
<210>53
   <211>34
   <212>DNA
   <213> Artificial Sequence
<220>
<223> Sequence of Mutant loxP
<400>53
   ataacttcgt ataatgtaac ctatacgaag ttat 34
<210>54
   <211>34
   <212>DNA
   <213> Artificial Sequence
<220>
   <223> Sequence of Mutant loxP
<400>54
   ataacttcgt ataatgtagt ctatacgaag ttat 34
<210>55
   <211>52
   <212>DNA
   <213> Artificial Sequence
<220>
<223> Sense Strand of Designed Sequence Containing Sequence of Wild-type loxP and Restriction Enzyme Recognition Site
<400>55
   tcgaggtgca cataacttcg tataatgtat gctatacgaa gttatacgcg tt 52
<210>56
   <211>52
   <212>DNA
   <213> Artificial Sequence
<220>
<223> Antisense Strand of Designed Sequence Containing Sequence of Wild-type loxP and Restriction Enzyme Recognition Site
<400>56
   ctagaacgcg tataacttcg tatagcatac attatacgaa gttatgtgca cc 52
<210>57
   <211>60
   <212>DNA
   <213> Artificial Sequence
<220>
   <223> Sense Strand of Designed Sequence Containing Sequence of Mutant loxP and Restriction Enzyme Recognition Site
<400>57
   tcgagtccgg aataacttcg tataacgtat actatacgaa gttatgctag catttaaatg 60
<210>58
   <211>60
   <212>DNA
   <213> Artificial Sequence
<220>
   <223> Antisense Strand of Designed Sequence Containing Sequence of Mutant loxP and Restriction Enzyme Recognition Site
<400>58
   tcgacattta aatgctagca taacttcgta tagtatacgt tatacgaagt tattccggac 60
<210>59
   <211>60
   <212>DNA
   <213> Artificial Sequence
<220>
   <223> Sense Strand of Designed Sequence Containing Sequence of Mutant loxP and Restriction Enzyme Recognition Site
<400>59
   cgcgcattta aattccggaa taacttcgta taacgtatac tatacgaagt tatgctagca 60
<210>60
   <211>60
   <212>DNA
   <213> Artificial Sequence
<220>
   <223> Antisense Strand of Designed Sequence Containing Sequence of Mutant loxP and Restriction Enzyme Recognition Site
<400>60
   cgcgtgctag cataacttcg tatagtatac gttatacgaa gttattccgg aatttaaatg 60
<210>61
   <211>10
   <212>DNA
   <213> Artificial Sequence
<220>
   <223> SwaI liner
<400>61
   gatttaaatc 10

## Description

This invention relates to a mutant loxP site and applications thereof. More particularly, the present invention relates to a mutant loxP site, in which a specific recombination between the mutant loxP site and wild-type loxP site cannot occur, but a specific recombination between mutant loxP sites can occur. The mutant loxP site may be used for gene manipulation or replacement.

### Background Art

It is not easy to integrate any gene into a specific site of the genome of an animal virus or a chromosome of an animal cell of the higher eukaryotes, or to delete a specific gene therefrom. A conventional method for gene integration into a specific site of a chromosome of an animal cell is to transform the cell with plasmid DNA. The plasmid comprises DNA designed for homologous recombination with the intended site of integration in the chromosome. The gene to be integrated is ligated within this DNA and the gene is thus integrated at the intended site by homologous recombination. The frequency of homologous recombination is, however, extremely low. To that end, the gene to be integrated and a drug resistance gene are usually integrated simultaneously, and successful transformants are selected by treatment with the relevant drug. Consequently, several months are required to obtain the transformed cells. Integration of a gene into the genome of an animal virus is slightly easier than integration into a chromosome of a cell. However it still involves various treatments, for example to construct recombinant adenoviruses, including homologous recombination using plasmids comprising the gene to be integrated, as well as cloning, selection and growth of the resulting recombinant virus (Bett et al., Proc. Natl. Acad. Sci., 91: 8802-8806, 1999 and Miyake et al., ibid. 93: 1320 - 1324, 1996).

One of the reasons why the gene manipulations described above are difficult is the low frequency of homologous recombination. By contrast, use of enzymes, for example restriction enzymes which can specifically recognize DNA sequences, in the construction of plasmids or bacteriophages can improve the efficiency of gene manipulation. An example of such an enzyme is recombinase Cre, derived from bacteriophage P1 of E. coli.

Cre is a specific DNA recombinase, which recognizes a specific nucleotide sequence (the loxP site) and conducts processes including DNA strand cleavage, strand exchange and ligation of each DNA strand within this site (Sternberg et al., J. Mol. Biol., 150: 467-468, 1981; Abremski et al., J. Biol. Chem., 259: 1509-1514, 1984; and Hoess et al., Proc. Natl. Acad. Sci., 81: 1026-1029, 1984). If two loxP sites of the same direction exist within the same DNA molecule, the DNA sequence between them is excised to form a circular DNA molecule (DNA excision reaction). If two loxP sites exist in different DNA molecules, one of which is a circular DNA, the circular DNA is inserted into the other DNA molecule through the loxP sites (insertion reaction). Although Cre and the loxP site were found in bacteriophage, the specific DNA recombination reaction is known to function not only in the prokaryotes but also in the eukaryotes, including animal cells, and in the animal viruses. Examples of excision reactions are seen in cultured animal cells (Sauer et al., Nucleic Acids Res., 17: 147-161, 1989 and Kanegae et al., Gene, 181: 207-212, 1996), animal viruses (Sauer et al., Proc. Natl. Acad. Sci., 85: 5166-5170, 1988; Anton et al., J. Virol., 69: 4600-4606, 1995; and Kanegae et al., Nucleic Acids Res., 23: 3816-3821, 1995), and transgenic mice (Lakso et al., Proc. Natl. Acad. Sci., 89: 6232-6236, 1992; Orban et al., ibid., 89: 6861-6865, 1992; Gu et al., Cell, 73: 1155-1164, 1993 and Gu et al., Science, 265: 103-106, 1994).

The insertion reaction can insert any gene into a chromosome of an animal cell or a viral genome if there are pre-existing loxP sites. However, the frequency of such insertions is extremely low (Fukushige et al., Proc. Natl. Acad. Sci., 89: 7905-79029, 1992 and Sauer et al., Proc. Natl. Acad. Sci., 84: 9108-9112, 1987), and consequently it is not practicable. This is because if two loxP sites exist in the same DNA molecule as a result of an insertion reaction, the excision reaction immediately occurs between these two sites. The insertion and excision reactions are reversible, but the reaction equilibrium lies overwhelmingly in favour of the excision reaction.

In order to increase the frequency of the insertion reaction, trials using a mutant type loxP site, which is different from the original nucleotide sequence of the loxP site (wild-type), were performed. The loxP site consists of DNA sequence of 34 bp. Among them, an 8 bp sequence between two 13 bp inverted repeats is designated as the spacer region. Recombination of the DNA strand is known to be carried out within the spacer region (Hoess et al., J. Mol. Biol., 181: 351-362, 1985).

| loxP site (34bp) | | |
|---|---|---|
| | 12345678 | |
| 5'-ATAACTTCGTATA | ATGTATGC | TATACGAAGTTAT-3' |
| 3'-TATTGAAGCATAT | TACATACG | ATATGCTTCAATA-5' |
| Inverted | Spacer | Inverted |
| Repeat | Region | Repeat |
| (13bp) | (8bp) | (13bp) |

It was shown that the specific DNA recombination reaction between a mutant loxP site, in which a base at position 7 in the spacer region is substituted from G (guanine) to A (adenine), and a wild-type loxP site cannot occur, but the specific DNA recombination reaction can occur between two mutant loxP sites (Hoess et al., Nucleic Acids Res., 14: 2287-2300, 1986).

Trials have used a DNA molecule with a gene of interest (the objective gene) located between a mutant loxP site and a wild-type loxP site. These trials examined whether the objective gene is inserted between a mutant loxP site and a wild-type loxP site in another DNA molecule, or whether the objective gene is replaced by whatever gene is between these sites on the other DNA molecule. Examples of these trials are replacement of a gene on a plasmid vector by a gene from a bacteriophage (Waterhouse et al., Nucleic Acids Res., 21: 2265-2266, 1993), insertion of a gene on a phagemid vector to a plasmid vector (Tsurushita et al., Gene, 172: 59-63, 1996) and replacement of a gene on a plasmid vector by a gene on a chromosome of an animal cell (Bethke et al., Nucleic Acids Res., 25: 2828-2834, 1997).

These trials were performed using only one mutant loxP site in each molecule, i.e. the mutant loxP site in which a base at position 7 of the spacer region was substituted from G (guanine) to A (adenine). Therefore it is unknown whether the mutant loxP site was preferable or not because the recombination reaction between the mutant loxP site and the wild-type loxP site does not occur. Further, in all three of the above trials, the experimental systems used a drug resistance gene as the objective gene, or a drug resistance gene together the objective gene. As a result, only recombinants which acquired drug resistance were amplified. Consequently, even if the efficiency of the actual gene insertion (gene replacement) is low due to the recombination between the loxP site with incomplete mutation and the wild-type loxP site, the experimental result is biased by the selection for drug resistance. The apparent reaction efficiency may therefore be too high.

Actually, as a result of direct and quantitative measurement , we have shown that a recombination reaction between the mutant loxP site (with substitution from G to A at position 7) and the wild-type loxP site occurs with a frequency of approximately 5%, which shows that mutation of the loxP site is incomplete.

As explained hereinabove, a technique for performing gene replacement in a chromosome of an animal cell using a mutant loxP site and a wild-type loxP site has been tried in the prior art, but its efficiency was not sufficient.

### Disclosure of Invention

An object of the present invention is to provide a mutant loxP site wherein, in the presence of recombinase Cre, recombination with a wild-type loxP site cannot occur, but recombination between two mutant loxP sites having the identical sequence can occur at the almost same efficiency of the recombination between two wild-type loxP sites. A further object of the present invention is to provide a method for gene integration or gene replacement with high efficiency in higher eukaryotes, including animal cells, using the combination of a wild-type loxP site and a mutant loxP site, or the combination of two mutant loxP sites having different sequences from each other. A further object of the present invention is to provide the application of such methods for gene transfer to animal and plant cells, construction of recombinant viruses, and gene manipulation in animal and plant bodies.

We have studied the mechanism of recombinase Cre-dependent recombination between two loxP sites and identified the nucleotide sequence of the loxP site which is essential for the reaction. This was done by preparing mutant loxP sites with a single base substitution for each of the 8 bases in the spacer region of the loxP site and studying their reactivity by means of a very sensitive assay method. Accordingly, we have identified, on the basis of these findings, mutant loxP sites, in which a recombination between two mutant loxP sites having the identical sequence can occur with nearly equal efficiency to the recombination between two wild-type loxP sites, and recombination between the mutant loxP sites and the wild-type loxP site or between mutant loxP sites having different sequences cannot occur. Further we have, as a result of combining these loxP sites, successfully integrated a gene with extremely high efficiency into a chromosome of an animal cell.

Accordingly, the present invention provides the following (1) - (21);
(1) A mutant loxP site having the following properties:
(a) a nucleotide sequence of the following formula derived from a wild-type E. coli P1 phage loxP site, wherein at least one of the bases consisting of the second (T), third (G), fourth (T) and fifth (A) bases, and at least one of the bases consisting of the sixth (T) and seventh (G) bases within the 8 bases in the central part (spacer region) of the sequence are substituted by different bases, and bases outside the spacer region are optionally substituted;

| | | |
|---|---|---|
| | 12345678 | |
| 5'ATAACTTCGTATA | ATGTATGC | TATACGAAGTTAT-3' |
| | Spacer Region | |

(b)a specific DNA recombination between said mutant loxP site and the wild-type loxP site cannot occur in the presence of recombinase Cre; and
(c)a specific DNA recombination between the mutant loxP sites having identical nucleotide sequences can occur in the presence of recombinase Cre.
(2) A mutant loxP site having the following properties:
(a) a nucleotide sequence of the following formula derived from a wild-type E. coli P1 phage loxP site, wherein a base selected from the group consisting of second (T), third (G) and fourth (T) bases of the spacer region is substituted by a different base, and bases in the region outside the spacer region are optionally substituted by any base;

| | | |
|---|---|---|
| | 12345678 | |
| 5'-ATAACTTCGTATA | ATGTATGC | TATACGAAGTTAT-3' |
| | Spacer Region | |

(b) a specific DNA recombination between said mutant loxP site and the wild-type loxP site cannot occur in the presence of recombinase Cre; and
(c) a specific DNA recombination between the mutant loxP sites having identical nucleotide sequences can occur in the presence of recombinase Cre.
(3) The mutant loxP site according to (1) or (2) above, wherein specific DNA recombination between the mutant loxP site and another mutant loxP site having a different nucleotide sequence cannot occur in the presence of recombinase Cre.
(4) The mutant loxP site according to (1) above, having the nucleotide sequence of SEQ ID NO: 26, SEQ ID NO: 29, SEQ ID NO: 30, SEQ ID NO: 35, SEQ ID NO: 39, SEQ ID NO: 42, or SEQ ID NO: 49.
(5) A DNA comprising the mutant loxP site according to any one of (1) to (4) above.
(6) A DNA comprising at least one wild-type loxP site and at least one mutant loxP site according to any one of (1) to (4) above.
(7) The DNA according to (6) above wherein a desired gene is inserted between the wild-type loxP site and the mutant loxP site.
(8) A DNA comprising at least two mutant loxP sites according to (1) or (2), wherein the two sites have different nucleotide sequences and wherein specific DNA recombination between the two sites cannot occur in the presence of a recombinase Cre.
(9) The DNA according to (8) above wherein a desired gene is inserted between two mutant loxP sites having different nucleotide sequences to each other.
(10) A non-human cell or an ex vivo human cell which is transformed by DNA according to any one of (6) to (9), provided that the human cell is not a germ cell and not an embryonic cell.
(11) A method of replacing gene A by a different gene B comprising reacting DNA (a) and DNA (b) in the presence of recombinase Cre:
wherein DNA (a) is a DNA comprising a wild-type loxP site, gene A and a mutant loxP site according to (1) or (2), in this order; and
DNA (b) is a circular DNA comprising a wild-type loxP site, a gene B and the same mutant loxP sequence as DNA (a), in this order; to obtain
DNA (c) in which gene A is replaced by gene B in DNA (a).

(12) A method for replacing a gene A by a different gene B comprising reacting DNA (a) and DNA (b) in the presence of recombinase Cre:
wherein DNA (a) is a DNA comprising two mutant loxP sites having different nucleotide sequences wherein specific DNA recombination between the two sites cannot occur in the present of a recombinase Cre (mutant loxP site 1 and mutant loxP site 2) and gene A, arranged in the order of mutant loxP site 1 / gene A / mutant loxP site 2;
DNA (b) is a circular DNA comprising the mutant loxP site 1, the gene B and the mutant loxP site 2, in this order;
to obtain DNA (c) in which gene A is replaced by gene B in DNA (a).

(13) The method according to (11) or (12) above wherein the gene B is not a functional gene.
(14) The method according to (11) or (12) above wherein the gene A is not a functional gene.
(15) The method according to any one of (11) to (14) above wherein DNA (a) is chromosomal DNA of a cell and DNA (b) is a plasmid DNA or DNA of a double-stranded circular DNA virus.
(16) The method according to any one of (11) to (14) above wherein DNA (a) is chromosomal DNA of a cell and DNA (b) has properties to be converted intracellularly to double-stranded circular DNA.
(17) The method according to any one of (11) to (14) above wherein DNA (a) is chromosomal DNA of a double-stranded DNA virus and DNA (b) is a plasmid DNA or DNA of a double-stranded circular DNA virus.
(18) The method according to any one of (11) to (14) above wherein DNA (a) is chromosomal DNA of a double-stranded DNA virus and DNA (b) has properties to be converted intracellularly to double-stranded circular DNA.
(19) The method according to (17) or (18) above wherein the double-stranded DNA virus of DNA (a) is adenovirus.
(20) A non-human transgenic animal having DNA according to any one of (6) to (9) above on a chromosome.
(21) A pharmaceutical product comprising DNA according to any one of (6) to (9) above.

### Brief Description of Drawings

Fig. 1: A structure of synthetic DNA containing wild-type loxP site. (s): sense strand. (a): antisense strand.
Fig. 2: Synthesized nucleotide sequences of the spacer region in mutant loxP sites with single-base substitutions (sense strand).
Fig. 3: Synthesized nucleotide sequences of the spacer region in mutant loxP sites with single-base substitutions (antisense strand).
Fig. 4: Synthesized sequences of the spacer region in mutant loxP sites with double-base substitutions (sense strand).
Fig. 5: Synthesized sequences of the spacer region in mutant loxP sites with double-base substitutions (antisense strand).
Fig. 6: A schematic drawing showing recombinase Cre dependent recombination between mutant loxP sites using linear DNA as substrate. "M": mutant loxP site. Arrow upper part of letter M: direction for loxP site. Numerals: length (bp) of fragment by BsaHI digestion.
Fig. 7: A schematic drawing showing a structure of plasmid pBRwt. "L": wild-type loxP site. Arrow upper part of letter L: direction for loxP site. ApR: ampicillin resistant gene. ori: replication origin in E. coli.
Fig. 8: A schematic drawing showing a linear DNA in which a wild-type loxP site at one end and a mutant loxP site at the other end are bound. "M": mutant loxP site. "L": wild-type loxP site. Arrow upper part of letter: direction for loxP site. (the same as in the figures hereinbelow). Arrow for downward direction: BsaHI site. Numerals show length (bp) of BsaHI digestion fragment.
Fig. 9: A schematic drawing showing a structure of plasmid pBLAmutant. Thick line: derived from pBR322. Thin line: derived from adenovirus.
Fig. 10: A schematic drawing showing recombinase Cre dependent recombination between wild-type loxP site and mutant loxP site using linear DNA as substrate. Thin arrow: DraI site.
Fig. 11: A schematic drawing showing plasmid puLwL (A), plasmid puLwM (B) and plasmid puMwL (C).
Fig. 12: A nucleotide sequence of mutant type synthetic DNA used for construction of plasmid puLwM. Underlined part: loxP site. Doubly underlined part: spacer region (8 bp). Thick letter: base substituted.
Fig. 13: A nucleotide sequence of mutant type synthetic DNA used for construction of plasmid puMwL. Underlined part: loxP site. Doubly underlined part: spacer region (8 bp). Thick letter: base substituted.
Fig. 14: A schematic drawing showing a structure of plasmid pCALwL (D) and plasmid pCALwM (E). CAPro: CAG promoter. GpA: β-globin poly(A) sequence.
Fig. 15: A schematic drawing showing a structure of plasmid puLZM (F) and plasmid puMOL (G). SpA: ori and poly(A) site of SV40.
Fig. 16: A schematic drawing showing a structure of plasmid puLZMOL (H) and plasmid puALZMOL (I). TpA: poly(A) sequence of thymidine kinase.
Fig. 17: Schematic drawing for experiment showing that lacZ gene on plasmid DNA (circular DNA) can be inserted onto adenovirus genome by combination of wild-type loxP site and mutant loxP site.
Fig. 18: A schematic drawing showing plasmid pCALwMds (A), plasmid pCALhmBMds (B) and plasmid pCALGFBMds (C). hmB: hygromycin B resistance gene. GFB: fusion gene with GFP and bleomycin resistance gene.
Fig. 19: Result of experiment on gene replacement on adenovirus genome. CV-1 cells were infected with Cre expression recombinant adenovirus at moi 15 and recombinant adenovirus for target at moi 9. Blue stained cells were observed for approximately 60% at moi 60 and approximately 90% at moi 100 of recombinant adenovirus for donor.
Fig. 20: Schematic drawing for experiment showing that lacZ gene on plasmid DNA (circular DNA) can be integrated onto chromosome of cells by combination of wild-type loxP site and mutant loxP site.
Fig. 21: Result of experiment on gene replacement on chromosome of cells. Six clones of hygromycin resistant target cells (C3, C7, C8, C11, C19 and C28) were infected for 1 hour with Cre expression recombinant adenovirus AxCANCre at moi 5 and recombinant adenovirus for donor AxALZMOL at moi 10, 30, 100 and 300, respectively. Blue stained cells were observed from 10% (C19) to 30% (C8), when cells were infected with recombinant adenovirus for donor at moi 100, although frequency was different as observed depending on cell lines.

### Best Mode for Carrying Out the Invention

The present invention is explained in detail as follows.

The mutant loxP site in the present invention is indicated by the nucleotide sequence, in which a specific base(s) in the spacer region of the wild-type loxP site is substituted by a base(s) different from the original base(s). As a result of such substitution, substrate specificity in the specific DNA recombination mediated by recombinase Cre is changed. The substitution may include substitution of other bases which do not affect substrate specificity. The substrate specificity means that, given three types of loxP site such as wild-type loxP site, mutant loxP site 1 and mutant loxP site 2, specific DNA recombination mediated by Cre can not substantially occur in combination with the wild-type loxP site and mutant loxP site 1, the wild-type loxP site and mutant loxP site 2, and the mutant loxP site 1 and mutant loxP site 2, respectively. The accuracy of substrate specificity is of a sufficient level to perform gene replacement as described hereinbelow.

The mutant loxP site and the wild-type loxP site are in advance integrated in a chromosome of a cell. The circular DNA, in which any gene is located between the same combination of two loxP sites, is then transduced into cells together with recombinase Cre. Then, an intermediate, in which four loxP sites are located, is generated as a result of the insertion reaction between the respective mutant loxP sites or the respective wild-type loxP sites. Subsequently, the excision reaction occurs between the loxP sites which did not perform the insertion reaction, (i.e. in case of the insertion reaction occurring between the wild-type loxP site, the excision reaction occurs between mutant loxP sites). Then the optional gene between the mutant loxP site and wild-type loxP site in the circular DNA is inserted between the mutant loxP site and the wild-type loxP site in the chromosome of the cell. In the case where another gene is located between the mutant loxP site and wild-type loxP site in the chromosome of the cell, this gene is excised from the chromosome by the above reaction and is replaced by the gene which is inserted in the circular DNA. Consequently, if mutant loxP site and wild-type loxP site exist in a chromosome of a cell, any gene can efficiently be inserted into the site between them. In addition, instead of a combination with mutant loxP site and wild-type loxP site, two different mutant loxP sites, between which specific DNA recombination does not occur, can be used.

The mutant loxP site of the present invention was identified as follows.

A mutant loxP site, in which a base at position 7 in the spacer region is substituted from G to A, is known as an example of mutant loxP site, in which substrate specificity of the loxP site is changed. (Hoess et al., Nucleic Acids Res., 14: 2287-2300, 1986). In that assay method, wild-type loxP site and mutant loxP site are ligated at both ends of the kanamycin resistance gene in a plasmid having an ampicillin resistance gene and a kanamycin resistance gene. E. coli expressing Cre is then transformed by the thus obtained plasmid. After selection of ampicillin resistant E. coli, the efficiency of the recombination between the wild-type loxP site and mutant loxP site was estimated by the existence of kanamycin resistance (kanamycin resistance is lost if recombination occurs between two loxP sites). It is uncertain that how much the actual recombination reaction is reflected by the results in such an experiment, because the result of recombination mediated by Cre in a single molecule of plasmid is amplified by several hundreds of millions of times since the experiment uses drug selection.

We have established a more direct and quantitative assay method to identify bases in the loxP site essential for recombination mediated by Cre. The assay is explained briefly as follows. DNA containing a wild type loxP site and DNA containing a mutant loxP site with a single-base substitution are synthesized by conventional methods. The DNA containing mutant loxP sites are made by substituting each of the 8 bases in the spacer region of wild type loxP one by one with a base selected from all substitutable bases (total 24 kinds of mutant loxP site). DNA fragments are made wherein a wild-type loxP site and a mutant loxP site are ligated at each end of a DNA fragment of suitable length (for example a linear DNA fragment prepared by digestion of plasmid pBR322 by restriction enzyme). The resulting DNA fragments are used as substrate. The substrates are reacted with Cre protein in an in vitro cell free system for recombination. The resulting products are then digested by restriction enzyme and electrophoresed to measure quantitatively the efficiency of recombination between the mutant loxP site and the wild-type loxP site. The following results are obtained.
(1) A mutant loxP site in which the base at positions 1 or 8 of the spacer region is substituted can react with a wild-type loxP site.
(2) The reaction between a wild-type loxP site and a mutant loxP site, in which the base at positions 2, 3, 4 or 5 is substituted, stops almost entirely at the intermediate stage without progressing to the final stage. The level of progress is slightly different depending on the substituted base position. In a mutant with substitution at position 5, a very small proportion proceeds to the final stage.
(3) The reaction between a wild-type loxP site and a mutant loxP site with substitution at position 6 produces almost no intermediate, and the reaction proceeds partially to the final stage. Progression to the final stage is higher than cases with substitution at position 5 or 7. The progression rate of the reaction depends on the kind of substituted base. In cases wherein an original base T is substituted by A, progression to the final stage is higher than for substitution by C or G.
(4) The reaction between a wild-type loxP site and a mutant loxP site with substitution at position 7, produces almost no intermediate, and a very small proportion of reactions proceeds to the final stage. The mutant loxP reported conventionally has a substitution of the base at position 7 from G to A. The reaction between a wild-type loxP site and a mutant loxP site with this substitution proceeds to the final stage at about 5% frequency. This frequency is too high for the gene replacement of the present invention. This is because, the recombination between the wild-type loxP site and the mutant loxP site occurs at a frequency as high as 5%, and therefore a gene between two loxP sites (wild-type and mutant type) may possibly be deleted by an excision reaction with Cre.

Intermediate as used herein means the condition of a so called Holliday-structure (Hoess et al. Proc. Natl. Acad. Sci., 84: 6840-6844, 1987), in which recombination in one strand of double stranded DNA occurs but the other strand does not. It is known that in the recombination reaction between two loxP sites, after cleavage and religation between bases at position 7 and 8 in the lower strand of the spacer region, the reaction is terminated by cleavage and religation between bases at position 1 and 2 in the upper strand (Gue et al., Nature, 389: 40-46, 1997). Consequently, substitution of bases at position from 2 to 5 is qualitatively different from the substitution of bases at position 6 and 7. It has become apparent that in the former, the recombination of the lower strand can occur but that of the upper strand cannot occur and the reaction stops at the intermediate stage In the latter, the recombination of the lower strand occurs partially with low frequency and the reaction partially proceeds up to the final stage.

Next, using the same assay method, recombination between two mutant loxP sites having identical nucleotide sequence is examined. Although differences in the efficiency of the reaction for different substitutions are observed, specific recombination mediated by Cre between the two loxP sites with a single-base substitution at any of positions 2 to 7 is confirmed. Differences in the efficiency of the reaction depend upon the kind of substituted base rather than the position of substitution. For example, substitution of the base at position 3 (G) by C, and that of the base at position 7 (G) by T are apparently worse for the efficiency of the reaction than substitution by the other 2 kinds of base.

Consequently, a mutant loxP site, in which a single base at position 2 (T), 3 (G) or 4 (T) in the 8 bases of the central part (spacer region) of the wild-type loxP site is substituted by another base, and DNA having the said mutant loxP site, are included within the present invention.

In addition, according to the above knowledge, a combination of two of the single base substitutions (a double-base substitution), which are qualitatively different to each other, i.e. a combination of a single base substitution at any one position from 2 to 5, with a substitution at either of positions 6 or 7, is expected to result in a mutant loxP site which has much higher specificity than a mutant loxP site with a single base substitution. Recombination efficiency between two identical mutant loxP sites with such double-base substitutions is expected to be almost equal to that for mutant loxP sites with a single base substitution.

Using the above described mutant loxP sites with double-base substitutions the efficiencies of recombination between a wild-type loxP site and each of the mutant loxP sites, and between two identical mutant loxP sites, were examined. The results showed that recombination between a wild-type loxP site and most of the mutant loxP sites with double-base substitutions cannot proceed entirely up to the final stage. Some mutant loxP sites cannot react with the wild-type loxP site at all. Consequently the substrate specificity of a double-base substitution mutant is obviously increased as compared with a single-base substitution mutant. However, the efficiency of the recombination between two identical mutant loxP sites is largely different depending on their substitutions. The following mutant loxP sites show practically sufficient recombination efficiency: T2C/G7A (#2171, SEQ ID NO: 26), T2A/G7A (#2271, SEQ ID NO: 29), T2A/G7C (#2272, SEQ ID NO: 30), G3A/G7A (#3171, SEQ ID NO: 35), G3T/G7A (#3371, SEQ ID NO: 39), T4C/G7A (#4171, SEQ ID NO: 42) and A5G/G7A (#5171, SEQ ID NO: 49). Among them, reaction efficiency of T2A/G7C (#2272) and A5G/G7A (#5171) is extremely high. Numerals show a position of substituted base. A base before the numeral shows a pre-substituted base and a base after the numeral shows a post-substituted base. For example, abbreviation T2C/G7A means that a base at position 2 to substituted from T to C, and a base at position 7 is substituted from G to A. Relationship between number and SEQ ID NO in a parenthesis is shown in Table 2 hereinafter.

As indicated, recombination between the wild type loxP site and a mutant loxP site, in which two bases in the spacer region of the wild-type loxP site are simultaneously substituted cannot occur, but the recombination reaction between two mutant loxP sites can occur with high efficiency. This shows that the substrate specificity of the mutant loxP site with double-base substitutions is higher than the loxP site with single-base substitution.

Consequently, the mutant loxP site, in which at least one base at position 2 (T), 3 (G), 4 (T) and 5 (A), and at least one base at position 6 or 7 in 8 bases in the central part (spacer region) of the wild type loxP are substituted by another base, and DNA having the said mutant loxP site are included in the present invention.

We have also examined whether the recombination between mutant loxP sites having different nucleotide sequences can occur or not. T2C/G7A (#2171) and T2A/G7C (#2272) are exemplified for that case. No recombination occurs between these two types of different mutant loxP sites. This fact means that at least three types of loxP sites having different substrate specificities, i.e. the wild-type loxP site, the mutant loxP site 1 and the mutant loxP site 2, are found and are preferable for gene replacement hereinbelow described.

A method for gene replacement using the mutant loxP site of the present invention and applications thereof are explained as follows.

The method for gene replacement of the present invention is comprised of: reacting DNA(a) and DNA(b) in the presence of recombinase Cre, wherein DNA (a) contains the wild-type loxP site, gene A and a mutant loxP site, in this order; and wherein DNA (b) is a circular DNA containing the wild-type loxP site, gene B and a mutant loxP site, in this order, thereby replacing gene A with gene B in DNA (a). In this case, recombination dependent on Cre between the wild-type loxP site and the mutant loxP site cannot occur, and gene A and gene B are any genes which are different in each other.

The method for gene replacement of the present invention is comprised of: reacting DNA(a) and DNA(b) in the presence of recombinase Cre, wherein DNA (a) contains two mutant type loxP sites each having different nucleotide sequences (mutant loxP site 1 and mutant loxP site 2) and gene A arranged in the order mutant loxP site 1 / gene A / mutant loxP site 2 in this order; and wherein DNA (b) is a circular DNA containing mutant loxP site 1, gene B and mutant loxP site 2, in this order; thereby replacing gene A with gene B in DNA (a).

In this case, recombination dependent on Cre between mutant loxP site 1 and mutant loxP site 2 cannot occur, and gene A and gene B are any genes which are different in each other.

The method for gene replacement will be performed basically as follows. The method using a wild-type loxP site and a mutant loxP site is explained, but a case using mutant loxP site 1 and mutant loxP site 2 is the same. Explanation will be given for the case of replacement of a gene in a chromosome of an animal cell. However the method of the present invention can also be applied for the genome of an animal virus, a plant cell, a chromosome of a microorganism such as yeast, bacteria or bacteriophage.

First of all, the wild-type loxP site and the mutant loxP site are in advance integrated into a chromosome of an animal cell. A gene A can optionally be located between the wild-type loxP site and the mutant loxP site. In this case gene replacement will be applied. If no gene A exists, then gene insertion will be applied.

A gene B to be introduced is inserted between two loxP sites in a circular DNA molecule, in which the wild-type loxP site and the mutant loxP site are inserted. The circular DNA molecule can be an already circularized molecule such as plasmid DNA or the DNA of a double stranded circular DNA virus, or a molecule which may be converted to circular molecule by any operation after transfection into cells. The circular DNA molecule, in which the gene B, the wild-type loxP site and the mutant loxP site are located is transfected into the cell previously mentioned by means of known method. Cre protein is simultaneously expressed in the cell, and then gene B in the circular DNA molecule is integrated between the wild-type loxP site and the mutant loxP site on the chromosome of the cell. If there is a gene A located between the two loxP sites on the chromosome of the cell, gene replacement, in which gene A is excised and gene B is inserted, occurs. If no gene is located between the two loxP sites on the chromosome, gene insertion occurs. Further, if no gene is located between the two loxP sites in the circular DNA, gene A on the chromosome can be excised. Furthermore, even if no gene A exists, since two lox P sites exist on the chromosome, another gene can be inserted between the two loxP sites by using another circular DNA molecule which has optional gene C between two loxP sites.

Conventional methods for transfection of circular DNA molecules in animal cells can be used. Examples are physicochemical methods such as electroporation, calcium phosphate transfection, DEAE-dextran transfection, lipofection and gene gun, and using a circular DNA virus. Examples of circular DNA viruses are papilloma virus and SV40. An example of a method for converting a DNA to a circular molecule after introduction into cells is a method using recombinase, examples of which are FLP derived from 2 µl plasmid of yeast, R derived from pSR1 plasmid of Zygosaccharomyces rouxii and Cre.

In order to integrate two loxP sites into a chromosome of an animal cell cells can be transformed by using plasmid DNA having two loxP sites. Transfection of the plasmid can be carried out by previously mentioned physicochemical methods. Further, viruses able to integrate viral genome into the chromosomes of cells, such as retrovirus, adeno-associated virus and HIV can also be used.

Methods for expressing Cre in animal cells are, for example, transfecting cells with DNA or RNA encoding the Cre gene, or introducing Cre protein itself into cells. Cells may be transfected with DNA or RNA encoding the Cre by a physicochemical method or a method using a virus vector. Examples of virus vectors are adenovirus, vaccinia virus, herpes virus and EB virus. Among them, adenovirus is preferable due to high efficiency of gene transfer.

The advantages of the gene replacement on a chromosome of an animal cell using a mutant loxP site of the present invention are high efficiency of the gene replacement and the possibility of insertion of the gene into a specific location on a chromosome. The latter is especially important for obtaining transformants. This is because, in methods of conventional transformation using DNA (except for homologous recombination) or virus vectors such as retrovirus and adeno-associated virus the objective gene is integrated into a chromosome at random. Therefore, the level of expression of the objective gene and its stability in the chromosome can vary a lot, depending on the integration site. Therefore, in order to obtain a transformed cell line, which can stably express the objective gene at high level for a long period, a large number of cell lines have to be screened and selected. Furthermore, the screening has to be repeated for each gene, i.e. each transformation. Contrary to that, in a method of the present invention, once a stable cell line has been obtained it is easy to insert any gene. High level expression of the gene between the two loxP sites can be used as an index of gene expression of any gene, It is therefore easy to obtain a stable cell line with high level expression of the gene introduced. Furthermore, since the efficiency is very high, no operation for drug selection, which is conventionally an essential operation, is required. The objective cell lines can be obtained within a short time using only cloning operations. Though there is no specific restriction of the cell type used, it is most preferable to use the method for the purpose of transformation of ES cells, used for the generation of transgenic animals.

The gene replacement on a chromosome of an animal cell according to the present invention can be applied for not only cultured cells but also the whole animal. As a method for expressing a specific foreign gene in an animal body, transgenic animals are known in the art. However, the generation of a transgenic animal which expresses an objective gene takes a long time. In order to generate transgenic animals by common methods, very complex operations are necessary, i.e. preparing ES cells for expressing objective gene, developing the ES cells in pseudopregnant maternal oviduct, screening the fetus by means of an index of expression of the objective gene to obtain transgenic animals, mating animals expressing objective gene. The operations require generally half a year to one year for each animal. Using the method of the present invention, once transgenic animals for gene transduction are generated, generation of transgenic animals corresponding to respective genes is not required. The transgenic animals for gene transduction are animals in which the mutant loxP site and the wild-type loxP site are integrated into a chromosome. The generation of such animals can be performed by the same method as in generation of conventional transgenic animals. Drug resistance genes such as neomycin resistance gene may be inserted between two loxP sites for drug selection. The gene can be inserted into a chromosome and expressed by transduction of tissues or cells with both (a) a circular DNA molecule, in which the objective gene is inserted between the wild-type loxP site and the mutant loxP site and (b) Cre protein. Introduction of (a) and (b) into the animal body can be sufficiently carried out by conventional methods such as liposome, virus vector and gene gun. Using the method of the present invention, transducing a different gene can be achieved using a circular DNA molecule (a) with the different gene. Generating such transgenic animals by common methods, which requires a long time, is no longer necessary. Further, by only transducing both of (a) and (b) locally, the objective gene can be inserted into only the target organs or tissues.

The present invention can be applied for the generation of a recombinant virus. An example of a virus is a DNA virus. Examples of DNA viruses are adenovirus, herpes viruses such as herpes simplex virus, EB virus, cytomegalovirus, and poxviruses such as vaccinia virus, canarypox virus and insect baculovirus. A further example is an RNA virus. Retrovirus is especially preferable. This is because, when preparing retrovirus vectors, potential virus producing cells are typically selected for particular retrovirus vectors, which produce particular genes. Using the present method, once high potential virus producing cell lines, which express a particular marker gene, are established, highly productive cell lines can easily be obtained by replacing the marker gene on the chromosome of the cell lines with the objective gene.

A concrete example of preparing a recombinant virus of the present invention is explained in the generation of a recombinant adenovirus. A conventional method for generating recombinant adenovirus is: transforming cells such as 293 cell by means of plasmid vector or cosmid vector, to which adenovirus genome and the objective gene are inserted. Then, after cloning the recombinant virus generated by homologous recombination, selecting the objective virus and proliferating the virus. This takes a long time. According to the method of the present invention, since the operation does not use low efficiency homologous recombination, the objective recombinant virus can be generated within a shorter time. Namely, at first, adenovirus for gene transduction, to which the mutant loxP site and the wild-type loxP site are inserted, is generated. Then the said virus is used to infect cells such as 293 cells (preferable for generation of recombinant adenovirus). Simultaneously, the plasmid DNA, to which the objective gene is inserted between the wild-type loxP site and the mutant loxP site, is transduced into said cells and Cre protein is expressed. As a result, the recombinant virus, to which the objective gene is inserted between the wild-type loxP site and the mutant loxP site, can be obtained with high frequency. In this case, the process may include generating an adenovirus for gene transduction in which the packaging signal is between the loxP sites and is deleted by recombination mediated by Cre. Plasmid DNA containing the objective gene and packaging signal is simultaneously added to enable selection of the objective virus. Cre protein may be transduced in the form of plasmid DNA, or supplied by cells which can express Cre protein constitutively or inducibly by any induction after cells are previously transformed.

The above method is explained in detail as follows. Explanation is given in the case of generating a recombinant adenovirus in which a foreign gene A is replaced by a foreign gene B. An example of a structure of a recombinant adenovirus for gene transduction is adenovirus in which loxP sites are inserted in the order of adenovirus left inverted terminal repeat (ITR) /wild-type loxP site / packaging signal / wild-type loxP site / gene A / mutant loxP site. A fragment comprising wild-type loxP site / gene A / mutant loxP site is also inserted into the E1 deletion region. An example of a plasmid DNA for insertion of foreign gene B is a plasmid having structure of wild-type loxP site / packaging signal / gene B / mutant loxP site. The adenovirus for gene transduction and the plasmid for foreign gene B insertion are simultaneously or sequentially transduced into cells such as 293 cells, which can express Cre protein. Then, the packaging signal between two wild-type loxP sites in the adenovirus is excised, and at the same time, the region containing the wild-type loxP site / gene A / mutant loxP site is replaced by the region containing the wild-type loxP site / packaging signal /gene B / mutant loxP site derived from the plasmid. Adenovirus in which gene replacement did not occur can replicate viral DNA, but cannot replicate virus itself, since the packaging signal is deleted by the action of a usual "excision reaction" having high reaction efficiency between two wild-type loxP sites. Accordingly DNA can not packaged within virus particles (virion). By contrast, gene replaced adenovirus has packaging signal, and consequently the adenovirus can replicate as virus and recombinant adenovirus containing "gene B" can be obtained with high frequency.

The insertion position of the mutant loxP site in the adenovirus can be proximal to the foreign gene A, or may be a position within the adenovirus genome distal to foreign gene A. Examples of position of insertion in the latter are the untranslated region between L3 gene and E2A gene, the deletion site of E3 gene and a region between upstream region of E4 gene and right ITR. If the mutant loxP site is inserted into any of these regions to perform gene replacement, the length of the DNA between the wild-type loxP site and mutant loxP site in the plasmid has to be adjusted, in order to ensure effective packaging of the generated adenovirus DNA into the virus particles (virion). However, since the gene replaced recombinant adenovirus has essential genes for virus replication deleted, adverse reactions which are problems of adenovirus may be reduced, for example when using it as a gene therapy vector.

DNA containing a mutant loxP site of the present invention can be used for gene therapy as a pharmaceutical. The method is explained as follows.

Firstly, the mutant loxP site and wild-type loxP site are in advance integrated into a chromosome of a human cell. This is achieved by administering DNA having a mutant loxP site and a wild-type loxP site , for example within a virus vector such as retrovirus or adeno-associated virus (AAV). AAV is preferably used, because a gene transferred by retrovirus could be integrated randomly into a chromosome, whereas with AAV, it is probable that the gene will be integrated into a specific region (AAV-S1 region in chromosome 19) with high frequency.. The viral gene encoded by AAV (Rep) is a prerequisite for gene integration into the above-mentioned specific chromosomal region. Since a large part of the AAV genome is deleted in the AAV vector presently used, the means for the specific integration mechanism are lost. However, as the length of sequence containing the mutant loxP site and the wild-type loxP site is less than 100 bp, it is possible to construct a virus vector, in which two loxP sites are inserted and the prerequisite viral gene is retained. The insertion site of the loxP sites is preferably just inside of the inverted terminal repeat (ITR) located at both ends of the AAV gene. As a result of administration of AAV containing two loxP sites to a human, two loxP sites can be integrated into a human chromosome.

Further, when a pharmaceutical preparation containing circular DNA, to which the objective gene is inserted between a wild-type loxP site and a mutant loxP site, and Cre protein or a DNA molecule encoding Cre gene are administered, then AAV gene between two loxP sites located in the chromosome is deleted and replaced by the objective gene. The circular DNA and the Cre protein or DNA molecule encoding Cre can be made by a method using vectors used for conventional gene therapy such as virus vectors or liposome vectors.

In the human cells which integrate the objective gene, the wild-type loxP site and mutant loxP site are located at both ends of the objective gene, and only the ITR of AAV is located outside loxP sites. Hence there is no structural gene of AAV, and consequently proteins derived from AAV do not express or become antigens. It is expected that the expression of the objective gene will continue stably for a long period. If in the future the integrated gene is no longer necessary, the gene can be deleted from the chromosome by administration of circular DNA which has no gene between the wild-type loxP site and the mutant loxP site. Further, if it becomes necessary to integrate a gene into the chromosome again, since two loxP sites remain on the chromosome, any gene can be integrated by the method described hereinbefore. As explained, DNA having a mutant loxP site of the present invention can be used as a pharmaceutical for gene therapy, by which integration of a gene into a chromosome and excision therefrom can freely be performed.

An example of a method for the transduction of the said DNA into cells is by means of a virus vector (Nikkei Science, April, 1994, pp 20-44, Monthly Pharmaceuticals, 36(1): 23-48, 1994, and cited references therein). Any suitable means for transduction can be used in the present invention.

Examples of transduction using virus vector involve inserting DNA containing a mutant loxP site of the present invention (or corresponding RNA) into viruses such as retrovirus, adenovirus, adeno-associated virus, herpes virus, vaccinia virus, poliovirus, RNA viruses (such as sindbis virus) and the like. Among them, methods using retrovirus, adenovirus and adeno-associated virus are most preferable.

Examples of other method for transduction are liposome, lipofectin, micro injection, calcium phosphate transfection, electroporation and gene gun. The liposome is most preferable.

The practical application of DNA having mutant loxP site as a pharmaceutical for use both in vivo (in which the said DNA is directly injected into body) and ex vivo (in which some cells are collected from the human body, the DNA is transduced into said cells in vitro, and said cells are then returned to the body), are known (Nikkei Science, 1994 April, pp 20-45, and Monthly Pharmaceuticals, 36(1): 23-48, 1994, and cited references therein). In the pharmaceuticals of the present invention, in vivo or ex vivo use can be selected and applied depending on the disease for treatment and the target organ.

For in vivo use, suitable administration routes are, for example, intravenously, intraarterialy, subcutaneously or intramuscularly, or direct administration into an organ such as kidney, liver, lung, brain or nerves.

For ex vivo use, conventional methods for collection of human cells (for example lymphocytes and hematopoietic stem cells) can be used, and the pharmaceuticals of the present invention are sensitized with them for gene transduction. The cells are then returned into the human body.

For in vivo use, various pharmaceutical formulations (for example, solutions) can be used, and generally injection containing DNA having a mutant loxP site as an active ingredient is applied. If necessary, conventional carrier can be added. Such the injections can be prepared by conventional methods. For example, DNA having a mutant loxP site can be dissolved in a suitable solution (for example, sterile water, buffer, saline, and the like), and then be filtered for sterilization before filling in aseptic vessels. Instead of DNA having a mutant loxP site, virus vector inserted with DNA containing a mutant loxP site can be formulated. Further, liposome (or HJV-liposome) encapsulated with DNA having mutant loxP site can be prepared as a formulation such as a suspension, freezing preparation or centrifuged concentrated freezing preparation.

The contents of the DNA having a mutant loxP site of the invention can be formulated depending upon the type of disease for treatment, target organ, age of patient and body weight. Administered doses are 0.0001 mg - 100 mg, preferably 0.001 - 10 mg, as with conventional DNA having mutant loxP site, and are administered once within several days to several months.

The above explained are methods of gene replacement using two loxP sites including a combination of a wild-type loxP site and mutant loxP site or a combination of mutant loxP site 1 and mutant loxP site 2. Application area for gene replacement can be extended by using more than three loxP sites having different substrate specificities. Examples of a combination of three loxP sites having different substrate specificities are the combination of wild-type loxP site and two mutant loxP sites or the combination of three mutant loxP sites. A method using a wild-type loxP site, a mutant loxP site 1 and a mutant loxP site 2 on a single DNA molecule is explained. DNA (a) and DNA (b) are reacted in the presence of recombinase Cre, wherein: DNA (a) comprises wild-type loxP site / gene A / mutant loxP site 1 / gene B / mutant loxP site 2, in this order, and wherein DNA (b) is a circular DNA comprising wild-type loxP site / gene C / mutant loxP site 1 in this order. Gene A in DNA (a) is then replaced by gene C. Alternatively, instead of the circular DNA (b), DNA (c) comprising mutant loxP site 1 / gene D /mutant loxP site 2 is reacted with DNA (a) in the presence of recombinase Cre. In this case gene B in DNA (a) is be replaced by gene D. Accordingly, it is possible to replace a desired gene among multiple genes in DNA (a) with an optional gene simply by using a different circular DNA.

Concrete examples of gene replacement using a mutant loxP site of the present invention are explained. In all following embodiments, the mutant loxP site, in which a base at position 2 in the spacer region is substituted from T to C and a base at position 7 is substituted from G to A (SEQ ID NO: 26), is used as the mutant loxP site.

### (1) Replacement of gene in adenovirus genome

The replacement gene in this example is E. coli lacZ gene. A lacZ gene on the circular DNA molecule is inserted between a wild-type loxP site and the mutant loxP site in the adenovirus genome. The effectiveness of the mutant loxP site of the present invention can be confirmed by this system.

Firstly, the adenovirus containing the gene to be replaced, i.e. the target adenovirus, was constructed. We constructed recombinant adenovirus (AxCALwM), in which CAG promoter / wild-type loxP site /mutant loxP site / poly(A) sequence was inserted in the left direction for transcription (the reverse direction of the transcriptional direction for the adenovirus E1 gene) in the E1 gene deletion site of adenovirus type 5, in which the E1 gene and E3 gene are deleted. The CAG promoter used herein is a high expression vector disclosed in Japanese Patent Unexamined Publication No. Hei 3-168087.

The circular DNA molecule, in which lacZ gene was inserted between the wild-type loxP site and the mutant loxP site, may optionally be transduced into cells directly by transfection with a plasmid DNA having the structure wild-type loxP site / lacZ gene / mutant loxP site. However, gene transfer efficiency into cells by transfection was only several tens percent, and the objective gene could not transducted into all cells. Consequently a method involving Cre-dependent intracellular formation of circular DNA using adenovirus vector was applied and as a result, circular DNA could be transduced into almost all cells. To that end, we constructed recombinant adenovirus (AxALZMOL), in which the poly(A) sequence of the thymidine kinase (TK) gene of herpes simplex virus / wild-type loxP site / lacZ gene / mutant loxP site / ori and poly(A) sequence of SV40 / wild-type loxP site were inserted into the E1 gene deletion site of adenovirus type 5 in the left direction for transcription. The thus constructed recombinant virus AxALZMOL is designated as the donor adenovirus. Recombination can occur between two wild-type loxP sites by an action of Cre protein on AxALZMOL, and a circular DNA molecule having a structure of wild-type loxP site / lacZ gene / mutant loxP site / ori and poly(A) sequence of SV40 was generated. The poly(A) sequence of the TK gene was inserted to prevent transcription of the lacZ gene from an unidentified promoter derived from adenovirus. Cre protein was supplied by using recombinant adenovirus AxCANCre expressing Cre (Kanegae et al., Nucleic Acids Res., 23: 3816-3821, 1995).

The gene replacement (insertion) experiment was conducted using the three recombinant adenoviruses described above. Adenovirus AxALXMOL as the donor, adenovirus AxCALwM as the target and adenovirus AxCANCre to supply Cre were simultaneously used to infect cultured animal cells (CV-1 cells or COS-1 cells). Both donor adenovirus AxALZMOL and the generated circular DNA molecule have the lacZ gene, but have no promoter. Consequently the lacZ gene cannot be expressed. The lacZ gene can only be expressed when the lacZ gene in the circular DNA molecule is inserted downstream of the CAG promoter in the target adenovirus AxCALwM, i.e. between the wild-type loxP site and the mutant loxP site. Consequently, expression of the lacZ gene is provides clear evidence for actual gene replacement (in this case, insertion).

No cells expressed the lacZ gene when infected with any of the above three viruses alone, or with combinations of any two viruses. The expression of the lacZ gene was observed clearly when cells were simultaneously infected with all three viruses. Furthermore the ratio of cells expressing the lacZ gene was increased depending on the amount of donor adenovirus, with a maximum of 90% of cells showing lacZ gene expression. These results showed that the lacZ gene on the donor adenovirus was inserted very efficiently between the wild-type loxP site and the mutant loxP site in the target adenovirus.

### (2) Replacement of a gene in a chromosome of an animal cell

The following experiments were conducted in order to show that a gene on a chromosome of an animal cell can also be replaced effectively. The principles of the experimental system are the same as in (1). In order to obtain cell lines (target cells) in which to replace a gene, CV-1 cells were transfected with the plasmid containing DNA bearing a structure of CAG promoter / wild-type loxP site / hygromycin B resistant gene / mutant loxP site / poly(A) sequence, then hygromycin resistant cell lines, inserted with only single copy of DNA bearing the above structure in a cell, were established.

These cell lines were simultaneously infected with donor adenovirus AxALZMOL and Cre expressing adenovirus AxCANCre. There were no lacZ gene expressing cells when the cells were infected with either adenovirus alone, but lacZ gene expressing cells were observed with high frequency when cells were doubly infected with both viruses. The proportion of lacZ gene expressing cells was different depending on the cell lines used, and lacZ gene were expressed in a maximum of approximately 30% of cells. Since cloning of cells did not occur in this experimental system, the proportion of cells expressing the lacZ gene shows directly the ratio of gene replacement on the chromosome. Consequently, the above result indicates that gene replacement on a chromosome of an animal cells can be performed with very high efficiency using the loxP site of the present invention.

The present invention will be explained in detail by showing examples. The present invention is not limited by these examples and conventional modification in the technical field of the present invention can naturally be applied. Various operations and treatments on phages, plasmids, DNA, E. coli and cultured cells in examples were performed, if not specified, according to methods described in "Molecular Cloning, A Laboratory Manual, T. Maniatis et al. Ed., 2nd Ed. (1989), Cold Spring Harbor Laboratory.

### Example 1

### Recombinase Cre dependent recombination reaction between two mutant loxP sites

### (1) Preparation of cell extract containing recombinase Cre

The following operations were performed in order to obtain cell extract containing Cre for use in a recombinase Cre dependent recombination reaction. 293 cells (cell line derived from human fetal kidney) in a 225 cm² flask were infected (37°C, 1 hour) with approx. 1 X 10⁹ PFU of Cre expressing recombinant adenovirus vector AxCANCre (Kanegae et al., Nucleic Acids Res., 23: 3816-3821, 1995). Medium (DMEM medium, containing 5% FCS) was further added thereto, and cultured for 24 hours. After cultivation, cell suspensions were centrifuged by low speed centrifuge at 1000 rpm for 5 minutes, and the cultured supernatant was discarded to collect cells. Cells were suspended by adding 5 ml of storage buffer [50% glycerol / 20 mM Tris-HCl (pH 7.5) /300 mM NaCl / 1 mM EDTA (pH 7.5)], sonicated using closed type sonicator at 200W for 2 minutes (30 sec. X 4) and released intracellular Cre protein. The thus obtained sonicated cell fluid was centrifuged by using micro centrifuge at 15000 rpm for 10 minutes, and the supernatant was stored in freezing (-80°C).

### (2) Preparation of synthetic DNA containing mutant loxP site

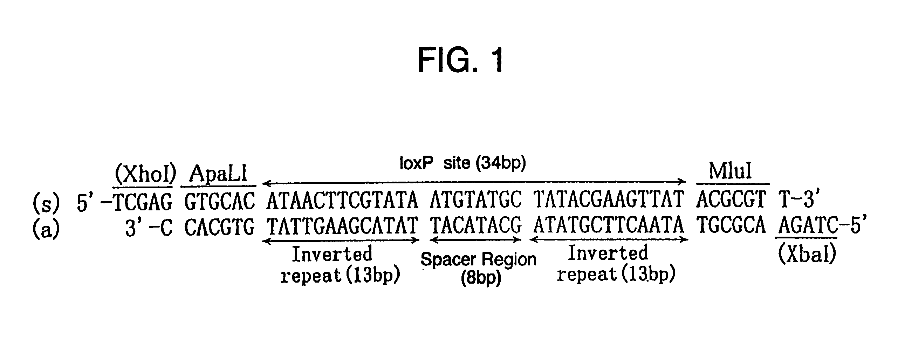

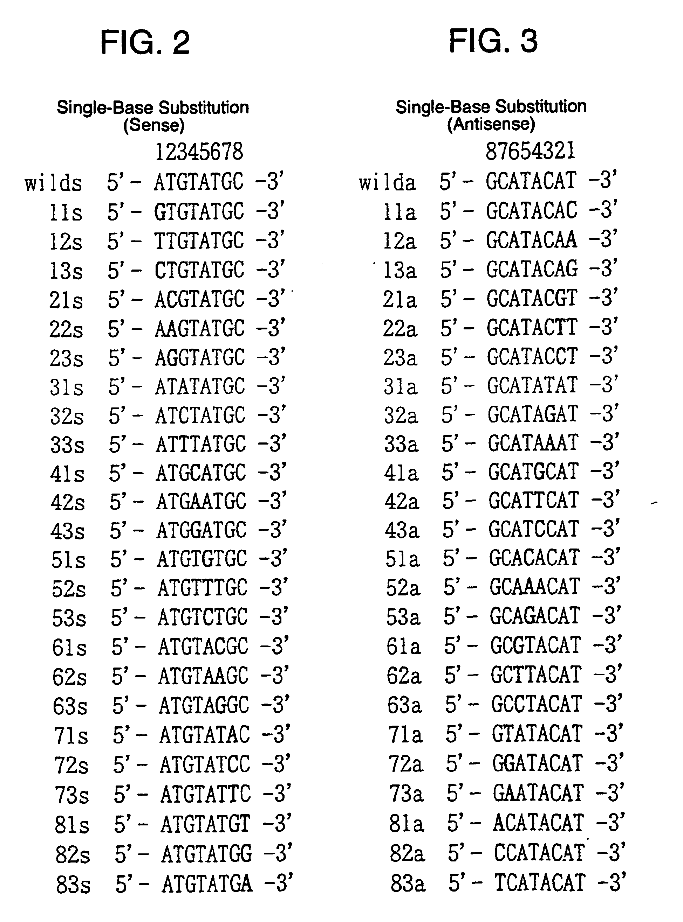

Synthetic DNAs, each consisting of 52 bases, in which 8 bases of the spacer region in the wild-type loxP site were substituted in one base or simultaneously two bases, were prepared. Types of the synthesized DNA were, 24 types of synthesized DNA were prepared with one base substituted, 29 types were prepared with two bases simultaneously substituted. The respective sense strand and antisense strand were synthesized. The DNA structure of a wild-type loxP site is shown in Fig. 1 (sense strand: SEQ ID NO: 55, antisense strand: SEQ ID NO: 56). The sequences of mutant loxP sites (sense strand and antisense strand) are shown in Fig. 2 - Fig. 5. The sense and antisense strands of the wild-type loxP site are not completely complementary sequences. These were designed such that when the strands are annealed to prepare double stranded DNA, 4 bases protrude at the 5' end of each strand to form the digestion fragments for restriction enzymes XbaI and XhoI. Accordingly, the double stranded DNA can be ligated with digestion fragments of restriction enzymes XbaI and NheI at the XbaI fragment end and digestion fragments of restriction enzymes XhoI and SalI at the XhoI fragment end, respectively.

All of the single stranded synthetic DNAs were kinased at 5' end with T4 polynucleotide kinase, and sense and antisense strands corresponding to respective mutations were annealed. These double stranded synthetic DNA are hereinafter designated as mutant loxP synthetic DNA.

### (3) Preparation of substrate DNA containing two mutant loxP sites

The following operations were conducted in order to obtain substrate DNA containing mutant loxP site with identical sequence in both ends of linear DNA.

Plasmid pBR322 was doubly digested with restriction enzymes NheI and SalI, and ligated with each of twenty-four mutant loxP synthetic DNA (single-base substitution) by T4 DNA ligase (plasmid : synthetic DNA, molar ratio 1 : 6) and digested doubly by restriction enzyme XbaI and XhoI. Mutant loxP synthetic DNA, which is bound with plurally both ends of pBR322 DNA, is deleted by the restriction enzyme treatment, and the linear DNA, approx. 4.1 kb, in which two mutant loxP synthetic DNAs, having identical sequence are ligated at the both ends of pBR322 DNA, is produced. Unreacted and restriction enzyme digested mutant loxP synthetic DNA were removed by using GEANCLEAN II (Funakoshi Inc.). The linear DNA (approx. 4.1 kb) ligated with mutant loxP site at the both ends is used in the following reaction as substrate DNA.

### (4) Cre dependent recombination reaction between two mutant loxP sites

It was examined whether a Cre dependent recombination reaction between two mutant loxP sites occurs using the following assay method.

Substrate DNA (1µg) prepared in (3) and cell extract containing Cre (10µl) prepared in (1) were incubated at 37°C for 30 min in a 50µl volume of buffer containing 50 mM Tris-HCl (pH 7.5) / 10 mM MgCl2 / 1 mg/ml bovine serum albumin / 1 mM phenylmethylsulfonyl fluoride (PMSF) / 5µg/ml aprotinin. After completion of the reaction, 45µl of TE buffer (pH 8.0) and 5µl of EDTA solution (pH 8.0) were added to the reaction mixture, extracted with phenol/ chloroform and with chloroform. Following ethanol precipitation, the recovered DNA was dissolved in 30µl of TE buffer (pH 8.0) containing RNase A (20µg/ml). Half volume thereof was digested with restriction enzyme BsaHI. The DNA bands detected by ethidium bromide (EtBr) staining after agarose gel electrophoresis were analyzed.

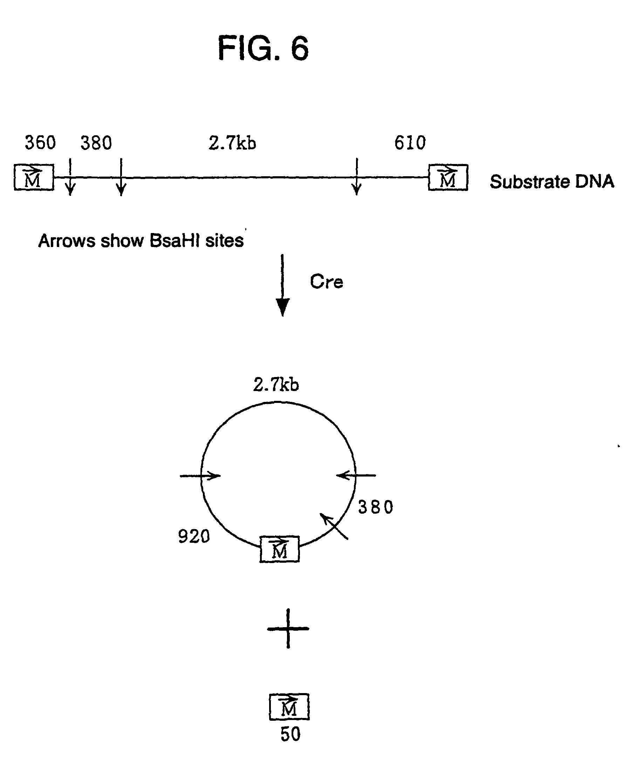

Before recombination mediated by Cre, restriction enzyme BsaHI digestion of linear substrate DNA (approx. 4.1 kb) generates four bands of 2.7 kb, 610 bp, 380 bp and 360 bp. On the other hand, as a result of Cre mediated recombination of substrate DNA, a circular DNA of approx. 4.0 kb containing one mutant loxP, and a linear DNA of approx. 50 bp consisting of only mutant loxP, are produced. Digestion of these DNA with restriction enzyme BsaHI generates four bands of 2.7 kb, 920 bp, 380 bp and 50 bp (refer to Fig. 6). Consequently, since band of 920 bp indicates that recombination mediated by Cre has occurred, and bands of 610 bp and 360 bp indicate that recombination mediated by Cre has not occurred, the ratio of the density of these bands indicates the efficiency of the recombination reaction. Results are shown in Table 1.

**Table 1 Cre-dependent Recombination Reactions between Wild-type loxP Site and Mutant loxP Site (Single Base Substitution)**

| SEQ ID NO. | Synthetic DNA NO | Mutant loxP Site - Mutant loxP Site | | Wild-type loxP Site - Mutant loxP Site | |
|---|---|---|---|---|---|
| | | Intermediate (970 bp) | Final Product (920 bp) | Intermediate (970 bp) | Final Product (920 bp) |
| 1 | wild | 1 | 9 | 1 | 9 |
| 2 | #11 | 4 | 2 | 3 | 5 |
| 3 | #12 | 6 | 3 | 4 | 7 |
| 4 | #13 | 4 | 3 | 3 | 7 |
| 5 | #21 | 4 | 5 | 5 | 0 |
| 6 | #22 | 4 | 5 | 7 | 0 |
| 7 | #23 | 6 | 5 | 9 | 0 |
| 8 | #31 | 5 | 7 | 9 | 0 |
| 9 | #32 | 6 | 3 | 9 | 0 |
| 10 | #33 | 6 | 6 | 9 | 0 |
| 11 | #41 | 1 | 7 | 9 | 0 |
| 12 | #42 | 5 | 6 | 9 | 0 |
| 13 | #43 | 5 | 6 | 9 | 0 |
| 14 | #51 | 1 | 9 | 7 | 1 |
| 15 | #52 | 1 | 9 | 7 | 2 |
| 16 | #53 | 1 | 8 | 7 | 1 |
| 17 | #61 | 1 | 8 | 1 | 3 |
| 18 | #62 | 1 | 8 | 1 | 6 |
| 19 | #63 | 1 | 9 | 1 | 2 |
| 20 | #71 | 1 | 7 | 1 | 1 |
| 21 | #72 | 3 | 7 | 1 | 1 |
| 22 | #73 | 3 | 1 | 1 | 1 |
| 23 | #81 | 3 | 3 | 4 | 7 |
| 24 | #82 | 8 | 5 | 5 | 9 |
| 25 | #83 | 2 | 9 | 2 | 8 |

In the table, amounts of reaction intermediates and final products are indicated in 10 grades, 0 - 9. A larger number indicates a larger amount of production. The amount of final reaction product (density of DNA bands) in the case of a reaction between two wild-type loxP sites was set as maximum numeral "9", In cases where no DNA band was detected (less than 5% of samples scoring "9"), the result was set as "0". Details of reaction intermediates will be explained in example 2-(2).

The recombination efficiencies between two identical mutant loxP sites #11 and between #73 were low. It was confirmed that the recombination between other respective identical mutant loxP sites mediated by Cre had occurred, although slight differences in the efficiency of recombination are observed depending on their sequences.

### Example 2

### Cre-dependent recombination between wild-type loxP site and mutant loxP site (No. 1)

### (1) Preparation of substrate DNA containing wild-type loxP site and mutant loxP site

### [1] Construction of plasmid (pBRwt) containing one wild-type loxP site

The following operations (a) and (b) were conducted in order to construct plasmid (pBRwt), by inserting one wild-type loxP site into plasmid pBR322.
(a) pBR322 was digested with restriction enzyme EcoNI, blunt-ended with Klenow enzyme and extracted with phenol/chloroform to denature EcoNI and Klenow enzyme, then the reaction mixture was substituted to TE buffer by gel filtration. EcoNI digested pBR322 was ligated with XhoI linker (5'-pCCTCGAGG-3'), doubly digested with restriction enzymes XhoI and PstI, then a DNA band of approx. 2.9 kb was excised from the agarose gel after electrophoresis. The DNA band was purified using GEANCLEAN II (Funakoshi Inc.) and the DNA fragment of approx. 2.9 kb having XhoI digested fragment in one end and PstI digested fragment in the other end was obtained.
(b) pBR322 was digested with restriction enzyme NheI, ligated with the synthetic DNA (52 bp) containing wild-type loxP site, and doubly digested with restriction enzymes XhoI and PstI. After agarose gel electrophoresis, the DNA band of approx. 1.4 kb containing a wild-type loxP site, was excised from the gel and purified using GEANCLEAN II (Funakoshi Inc.). The DNA fragment of approx. 1.4 kb, has also a XhoI digested fragment in one end and a PstI digested fragment in the other end.

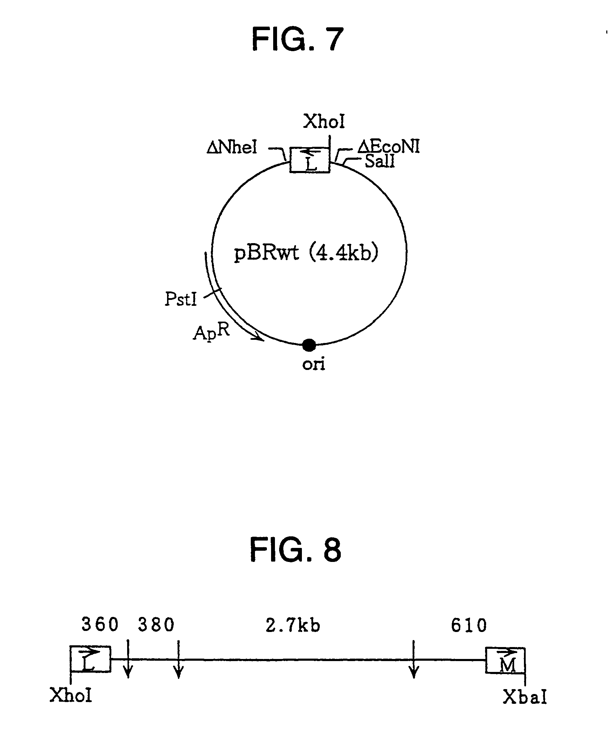

Both of the DNA prepared in (a) and (b) were ligated. E. coli was then transformed and plasmid pBRwt (4.4 kb, Fig. 7)obtained, in which one wild-type loxP site was inserted between NheI site and EcoNI site of pBR322.

### [2] Preparation of substrate DNA containing wild-type loxP site and mutant loxP site

The following operations were conducted in order to prepare substrate DNA having a wild-type loxP site in one end and a mutant loxP site in the other end of a linear DNA.

SalI site was located in a position about 30 bp distance from wild type loxP site in plasmid pBRwt, pBRwt was digested with restriction enzyme SalI to prepare linear DNA. SalI digested pBRwt was ligated with the mutant loxP synthetic DNA (52 bp, 24 kinds), which was the same DNA as of example 1-(3), (plasmid: synthetic DNA, molar ratio 1:6). As a result of this operation, XhoI digested fragment side of the mutant loxP synthetic DNA was ligated to SalI digested site of pBRwt. The reaction mixture was doubly digested with restriction enzymes XbaI and XhoI to remove mutant loxP sites which were ligated multiple with both ends of pBRwt, further the unreacted and restriction enzyme digested mutant loxP synthetic DNA were removed from the reaction mixture using GEANCLEAN II (Funakoshi Inc.). The linear DNA (approx. 4.4 kb, Fig. 8), in which a wild-type loxP site in one end and a mutant loxP site in the other end are ligated to each other, was obtained. This DNA was used in the following reaction as a substrate DNA.

### (2) Cre-dependent recombination between wild-type loxP site and mutant loxP site

Reaction and analytical method are the same as in example 1-(4), with the proviso that the substrate DNA used in this experiment is the DNA shown in example 2-(1) in place of the DNA shown in example 1-(3).

In the nucleotide sequence of the wild-type loxP site shown hereinbelow, it is known that the recombination between two loxP sites is at first initiated with cleavage and religation between bases at position 7 and 8 in the lower DNA strand of the 8-bp spacer region, then terminated by cleavage and religation between bases at position 1 and 2 in the upper DNA strand. (Gue et al. Nature, 389: 40-46, 1997).

| loxP site (34bp) | | |
|---|---|---|
| | | |
| | 12345678 | |
| 5'-ATAACTTCGTATA | ATGTATGC | TATACGAAGTTAT-3' |
| 3'-TATTGAAGCATAT | TACATACG | ATATGCTTCAATA-5' |
| Inverted | Spacer | Inverted |
| Repeat | Region | Repeat |
| (13bp) | (8bp) | (13bp) |

Consequently, if the recombination in the lower DNA strand occurs but the recombination in the upper DNA strand does not occur, the result is detected as an intermediate in the reaction. The intermediate was detected as a DNA band of approx 970 bp in the assay system used by us.

As previously shown in example 1-(4), the DNA band of 920 bp indicates that recombination in both of upper and lower DNA strands completed, and the DNA band of 970 bp indicates that the reaction has stopped in the intermediate stage.

Table 1 shows the result of analysis, for which existence of these two bands was specifically noticed. Outlines of result in table 1 are shown hereinbelow (numerals after # are SEQ ID NO of mutant loxP site).
#11, 12, 13 (SEQ ID NO: 2, 3, 4): reaction proceeded to final stage.
#21, 22, 23 (SEQ ID NO: 5, 6, 7): reaction stopped in the intermediate and did not proceed to the final stage.
#31, 32, 33 (SEQ ID NO: 8, 9, 10): reaction stopped in the intermediate and did not proceed to the final stage.
#41, 42, 43 (SEQ ID NO: 11, 12, 13): reaction stopped in the intermediate and did not proceed to the final stage.
#51, 52, 53 (SEQ ID NO: 14, 15, 16): reaction stopped almost in the intermediate and very small portion (about
5%) proceeded to the final stage.
#61, 63 (SEQ ID NO: 17, 19): almost no intermediate was produced and very small portion (5-10%) of reaction proceeded to the final stage.
#62 (SEQ ID NO: 18): almost no intermediate was produced and a portion (5-10%) of reaction proceeded to the final stage (amount of the final product was more than #61 and 63) .
#71, 72, 73 (SEQ ID NO: 20, 21, 22): almost no intermediate was produced and very small part of reaction proceeded to the final stage (about 5%, final products was less than #61 and 63).
#81, 82, 83 (SEQ ID NO: 23, 24, 25): Reaction proceeded to the final stage.

In conclusion, the substitution effect of bases at position from 1 to 8 are summarized according to each substituted position as follows:
(1)The mutant loxP site with substitution at position 1 or 8 reacts with wild type loxP site;
(2)The mutant loxP site with substitution at the position 2, 3 or 4 hardly react with wild type loxP site;
(3)The mutant loxP site with substitution at position 5, 6 or 7 is difficult to react with wild type loxP site, but reacts in part. These results indicated that substitution of bases at positions from 2 to 5 was qualitatively different from the substitution of bases at positions 6 and 7, and that in the former, the reaction stopped at the intermediate stage, and in the latter, the first step of the reaction was markedly inhibited.

Further, according to the result of this assay method, it has become clear that the mutant loxP site corresponding to #71, which was reported to undergo no specific DNA recombination with the wild-type loxP site (Hoess et al., Nucleic Acids Res., 14: 2287-2300, 1986), did not lose reactivity with the wild-type loxP site, and recombination occurs at about 5% of frequency.

### Example 3

### Cre-dependent recombination between wild-type loxP site and mutant loxP site (No. 2)

### (1) Preparation of substrate DNA containing a wild-type loxP site and a mutant loxP site

The following operations were conducted in order to obtain substrate DNA used for detecting the Cre-dependent recombination between wild-type loxP site and mutant loxP site by a more sensitive assay system than the assay system used in examples 1 and 2.

### [1] Construction of plasmid (pBLAmutant) containing a wild-type loxP site and a mutant loxP site

The following operations were conducted in order to construct the plasmid, in which a DNA fragment derived from adenovirus type 5 was ligated with a linear DNA fragment of approx. 4.4 kb as prepared in example 2-(1)-[2])..

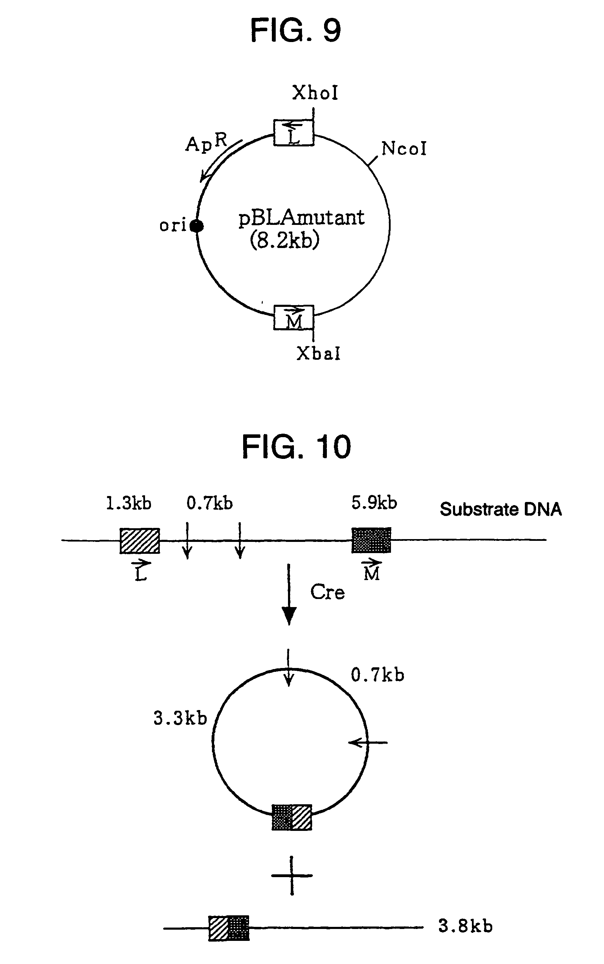

A cosmid vector pAxcw, to which almost full length DNA fragment of adenovirus type 5 except for E1 and E3 genes was inserted (Japanese Patent Unexamined Publication No. Hei 8-308585, page 15), was doubly digested with restriction enzymes XbaI and XhoI. The digested reaction mixture was subjected to agarose gel electrophoresis and a DNA band of approx. 3.8 kb, was excised from the gel, then purified using GEANCLEAN II (Funakoshi Inc.). This DNA fragment of 3.8 kb was ligated with the linear DNA fragment (approx. 4.4 kb) prepared in Example 2(1)-[2], in which a wild-type loxP site was ligated in one end and a mutant loxP site was ligated in the other end. E. coli was then transformed and plasmid pBLAmutant (8.2 kb, Fig. 9) was obtained. The plasmid pBLAmutant is a general term for plasmids treated by the above operation. Actually, plasmids having one of mutant loxP site corresponding to #11, #21, #22, #23, #31, #41, #51, #61, #71, #71, #73 or #81 in table 1 and a wild-type loxP site were constructed.

### [2] Preparation of substrate DNA containing a wild-type loxP site and a mutant loxP site

The following operations were conducted in order to prepare linear DNA fragment containing a wild-type loxP site and a mutant loxP site from plasmid pBLAmutant, which was used as substrate for Cre-dependent recombination.

Plasmid pBLAmutant, in which a unique restriction enzyme NcoI site was located in the region of the inserted adenovirus genome, was digested with restriction enzyme NcoI to prepare linear DNA fragment. The digested reaction mixture was subjected to agarose gel electrophoresis, and a DNA band of approx. 8.2 kb was excised from the gel and purified using GEANCLEAN II (Funakoshi Inc.). This linear DNA of 8.2 kb, was used as substrate in the following reaction.

### (2) Cre-dependent recombination between a wild-type loxP site and a mutant loxP site

To examine the efficiency of recombination mediated by Cre, the above described substrate DNA and the reaction mixture shown in example 1-(4), were incubated at 37°C for 30 minutes. After completion of the reaction, 45µl of TE buffer (pH 8.0) and 5µl of EDTA solution (pH 8.0) were added to the reaction mixture, extracted with phenol/chloroform and with chloroform. Following ethanol precipitation, the recovered DNA was dissolved in 30µl of TE buffer (pH 8.0) containing RNase A (20µg/ml). Half volume thereof was digested with restriction enzyme DraI. The product was subjected to agarose gel electrophoresis, stained with ethidium bromide (EtBr), and detected DNA bands were analyzed.

In the linear DNA fragment used for substrate, since two DraI sites are located between wild-type loxP site and mutant loxP site, if recombination mediated by Cre does not occur, three bands, 5.9 kb, 1.3 kb and 0.7 kb, are generated by DraI digestion (Fig. 10). Contrary to that, if recombination of substrate DNA mediated by Cre does occur, circular DNA of approx. 4 kb containing two DraI sites and one loxP site, and linear DNA fragment of approx. 3.8 kb containing one loxP site and no DraI site, are generated. Digestion of these DNA with DraI generates three bands of 3.8 kb, 3.3 kb and 0.7 kb. Consequently, existence of bands of 3.8 kb and 3.3 kb indicates that recombination mediated by Cre has occurred, and existence of bands of 5.9 kb and 1.3 kb indicates that recombination mediated by Cre has not occurred. The ratio of the density of these bands indicates the efficiency of the recombination reaction. Further, the reaction intermediate explained in example 2-(2) was detected as a DNA band of several tens kb in the present assay. Results are summarized as follows. #11, 81: reaction proceeded to the final stage;
#21, 22, 23, 31, 41: reaction stopped in the intermediate and did not proceed to the final stage;
#51: reaction stopped almost entirely in the intermediate, but very small portion of reaction proceeded to the final stage;
#61: almost no intermediate was produced, but some portion of reaction proceeded to the final stage;
#71: almost no intermediate was produced, but some portion of reaction proceeded to the final stage (amount of final product was more than #72, 73); and
#72, 73: almost no intermediate was produced, but very small portion of reaction proceeded to the final stage.

According to the above results, it was found that the tendency of the reaction was the same when the more sensitive assay method was used. Further, even in the mutant loxP site (corresponding to #71), which was reported to undergo no specific DNA recombination with the wild-type loxP site (Hoess et al., Nucleic Acids Res., 14: 2287-2300, 1986), reactivity with the wild-type loxP site was still retained in some part. Furthermore, the level of reactivity of #71 (amount of final product) was higher than that of #72 and #73, which were substituted at the same position, and it was reconfirmed that the substitution in #71 was insufficient to completely lose reactivity with wild-type loxP site.

### Example 4

### Cre-dependent recombination between mutant loxP sites with double-base substitutions, and wild-type loxP site

### (1) Cre-dependent recombination between wild-type loxP site and mutant loxP site

### [1] Preparation of substrate DNA containing a wild-type loxP site and a mutant loxP site

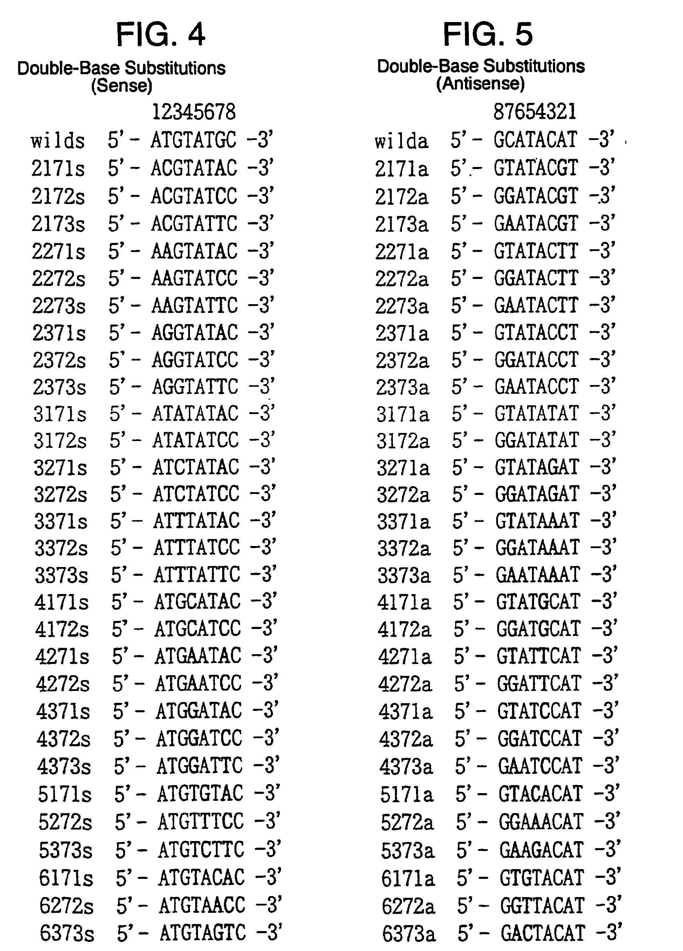

Substrate DNA having wild-type loxP site at one end and mutant loxP site at the other end in a linear DNA fragment was prepared according to the method shown in example 2-(1)-[2]. Plasmid pBRwt is a plasmid pBR322, in which a wild-type loxP site was inserted (described in example 2-(1)-[1]). pBRwt was digested with SalI and ligated with each mutant loxP site with double-base substitutions (refer to Fig. 4 and Fig. 5). The resulting linear DNA fragments were use as substrate DNA.

### [2] Cre-dependent recombination (No. 1)

According to the method as shown in example 1-(4), the efficiency of Cre-dependent recombination was examined using the substrate DNA prepared in [1]. Results are shown in Table 2 (right column).

**Table 2 Cre-dependent Recombination Reactions between Wild-type loxP Site and Mutant Type loxP Site (Double-Base Substitutions)**

| SEQ ID NO. | Synthetic DNA NO | Mutant loxP Site - Mutant loxP Site | | Wild-type loxP Site - Mutant loxP Site | |
|---|---|---|---|---|---|
| | | Intermediate (970 bp) | Final Product (920 bp) | Intermediate (970 bp) | Final Product (920 bp) |
| 1 | wild | 1 | 9 | 1 | 9 |
| 20 | #71 | 1 | 6 | 0 | 1 |
| 26 | #2171 | 3 | 3 | 0 | 0 |
| 27 | #2172 | 4 | 2 | | |
| 28 | #2173 | 6 | 0 | | |
| 29 | #2271 | 2 | 6 | | |
| 30 | #2272 | 1 | 6 | 0 | 0 |
| 31 | #2273 | 3 | 4 | | |
| 32 | #2371 | 3 | 2 | | |
| 33 | #2372 | 2 | 0 | | |
| 34 | #2373 | 6 | 1 | 0 | 0 |
| 35 | #3171 | 4 | 7 | 0 | 0 |
| 36 | #3172 | 0 | 0 | | |
| 37 | #3271 | 3 | 4 | | |
| 38 | #3272 | 6 | 2 | 0 | 0 |
| 39 | #3371 | 5 | 5 | | |
| 40 | #3372 | 6 | 1 | | |
| 41 | #3373 | 6 | 2 | 0 | 0 |
| 42 | #4171 | 0 | 4 | 0 | 0 |
| 43 | #4172 | 2 | 4 | | |
| 44 | #4271 | 4 | 2 | | |
| 45 | #4272 | 4 | 1 | 0 | 0 |
| 46 | #4371 | 4 | 2 | | |
| 47 | #4372 | 3 | 2 | | |
| 48 | #4373 | 7 | 0 | 1 | 0 |
| 49 | #5171 | 0 | 6 | 0 | 0 |
| 50 | #5272 | 1 | 4 | 0 | 0 |
| 51 | #5373 | 1 | 2 | 0 | 0 |
| 52 | #6171 | 0 | 6 | 1 | 1 |
| 53 | #6272 | 1 | 2 | 0 | 0 |
| 54 | #6373 | 1 | 2 | 0 | 0 |

In table 2, amounts of reaction intermediates and final products were indicated in 10 grades from 0 - 9 as described for table 1.

When mutant loxP site #6171 was used, the final product (920 bp) was confirmed partially. This result showed that recombination with the wild-type loxP site occurred. However, other mutant loxP sites analyzed did not react entirely with wild-type loxP site, and the reactivity was lower than the case of mutant loxP site with single-base substitution.

### [3] Construction of plasmid containing wild-type loxP site and mutant loxP site

Three types of plasmid pBLAmutant (8.2 kb, refer to Fig. 9) containing both wild-type loxP site and mutant loxP site were constructed from mutant loxP synthetic DNA of #2171, #2272 and #2373, according to a method shown in examples 2-(1)-[2] and 3-(1)-[1].

### [4] Cre-dependent recombination (No. 2.)

After the plasmid constructed in [3] was digested with NheI, linear substrate DNA fragments (8.2 kb) containing wild-type loxP site and a mutant loxP site were prepared according to the method shown in example 3-(1)-[2]. According to the method show in example 3-(2), the efficiencies of Cre-dependent recombination were examined using these substrate DNAs. With all three types of mutant loxP site, neither the reaction intermediates nor the final products were produced. Thus, it was shown that mutant loxP sites with double-base substitutions do not undergo recombination with a wild-type loxP site, with the reaction specificity further increased as compared with a mutant loxP site with single-base substitution.

### (2) Cre-dependent recombination between two identical mutant loxP sites

### [1] Preparation of substrate DNA containing two mutant loxP sites

For all 29 types of mutant loxP site (refer to Fig. 4 and Fig. 5), substrate DNAs containing an identical mutant loxP site at both ends of a linear DNA fragment were prepared according to the method shown in example 1-(3). The above mutant loxP synthetic DNAs were ligated with plasmid pBR322 which was doubly digested with restriction enzymes NheI and SalI, and linear DNA fragments of approx. 4.1 kb having two identical mutant loxP sites at both ends were obtained.

### [2] Cre-dependent recombination (No. 1)

According to the method shown in example 1-(4), the efficiencies of Cre-dependent recombination were examined using the substrate DNAs prepared in [1]. Results are shown in Table 2 (left column). Though there were mutant loxP sites for which recombination did not occur, for example #2173 and #2372, it was confirmed that recombination occurs in #2271, #2272, #3171, #3371, #4171, #5171 and #6171 with almost same efficiency as in the mutant loxP site with single-base substitution, and in #2171, #2273, #3271 and #4172at about half the level of efficiency in the mutant loxP site with single-base substitution.

### [3] Construction of plasmid containing one mutant loxP site

Using mutant loxP synthetic DNA of #2171, #2272 and #2373, three types of plasmids (pBR2171, pBR2272 and pBR2373, 4.4 kb, respectively, refer to Fig. 7), to which a mutant loxP site was inserted between NheI site and EcoNI site of plasmid pBR322, were constructed according to example 2-(1)-[1].

### [4] Construction of plasmid containing two identical mutant loxP sites

Three types of plasmids (pBR2171, pBR2272 and pBR2373) constructed in [3] were digested with restriction enzyme SalI and ligated with mutant loxP synthetic DNA (#2171, #2272 and #2373) having the same sequence mutant loxP site as the plasmid, respectively. Three types of plasmid (pBLA2171x2, pBLA2272x2 and pBLA2373x2, each 8.2 kb, refer to Fig. 9) containing two identical mutant loxP sites were thus obtained, according to methods shown in examples 2-(1)-[2] and 3-(1)-[1].

### [5] Cre-dependent recombination (No. 2)

The three types of plasmids constructed in [4] were digested with restriction enzyme NheI and linear substrate DNA (8.2 kb) containing two identical mutant loxP sites were prepared according to methods shown in example 3-(1)-[2]. According to the method shown in example 3-(2), the efficiencies of Cre-dependent recombination were examined using these substrate DNAs. The results showed that the efficiency of recombination between two mutant loxP sites #2272 was as high as the efficiency of recombination between the wild-type loxP sites. In the mutant loxP site #2171, the reaction proceeded up to final stage, although in some portion reaction stopped in the intermediate. In the mutant loxP site #2373, almost all reactions stopped in the intermediate and did not proceed to the final stage.

### (3) Examination on Cre-dependent recombination between mutant loxP sites with different sequence

### [1] Construction of plasmid containing mutant loxP sites with different sequence

Plasmids, pBR2171 and pBR2272 constructed in (2)-[3] were digested with restriction enzyme SalI and ligated with mutant loxP synthetic DNA with different sequence inserted in the plasmid (pBR2171 was ligated with #2272 or #2373 and pBR2272 was ligated with #2373). Three types of plasmids (pBLA2171-2272, pBLA2171-2373 and pBLA2272-2373, each 8.2 kb, refer to Fig. 4) containing two mutant loxP sites with different sequence were thus obtained according to methods shown in examples 2-(1)-[2] and 3-(1)-[1].

### [2] Cre-dependent recombinant

The plasmid constructed in [1] were digested with restriction enzyme NheI, and the linear substrate DNAs (8.2 kb) containing two mutant loxP sites with different sequence were prepared according to method shown in example 3-(1)-[2]. Using this substrate DNA, the efficiency of Cre-dependent recombination was examined according to the method shown in example 3-(2). The results showed that among all combinations of three mutant loxP sites with different sequence (#2171, #2272 and #2373), no Cre-dependent recombination occurred. That is, it was shown that each mutant loxP site could not cross react with other mutant loxP site.

The results from (1) to (3) show that the recognition specificity of the mutant loxP site with double-base substitutions was higher than that of the mutant loxP site with single-base substitution. That is, mutant loxP sites with double-base substitutions could not react with either wild-type loxP sites or mutant loxP sites with different sequences. In addition, it was shown that the recombination efficiency between identical mutant loxP sites was as same as the recombination efficiency between wild-type loxP sites. Further, it was shown that the position of substitution was preferably in combination between one base substitution at position 2, 3, 4 or 5 of the spacer region, and one base substitution at position 7 of the spacer region. Even when the position(s) of substitution are the same, reactivity and reaction specificity are different depending on a kind of base to be substituted. Thus, preferred examples of the mutant loxP site used in the present invention are: #2171 (SEQ ID NO: 26), #2271 (SEQ ID NO: 29), #2272 (SEQ ID NU: 30), #3171 (SEQ ID NO: 35), #3371 (SEQ ID NO: 39), #4171 (SEQ ID NO: 42), and #5171 (SEQ ID NO: 49).

### Example 5

### Construction of plasmid and cosmid vector required for replacement of gene inserted between wild-type loxP site and mutant loxP site

### (1) Construction of plasmid and cosmid vector for target in gene replacement

### [1] Construction of plasmid (puLwL) having cloning site between two wild-type loxP sites

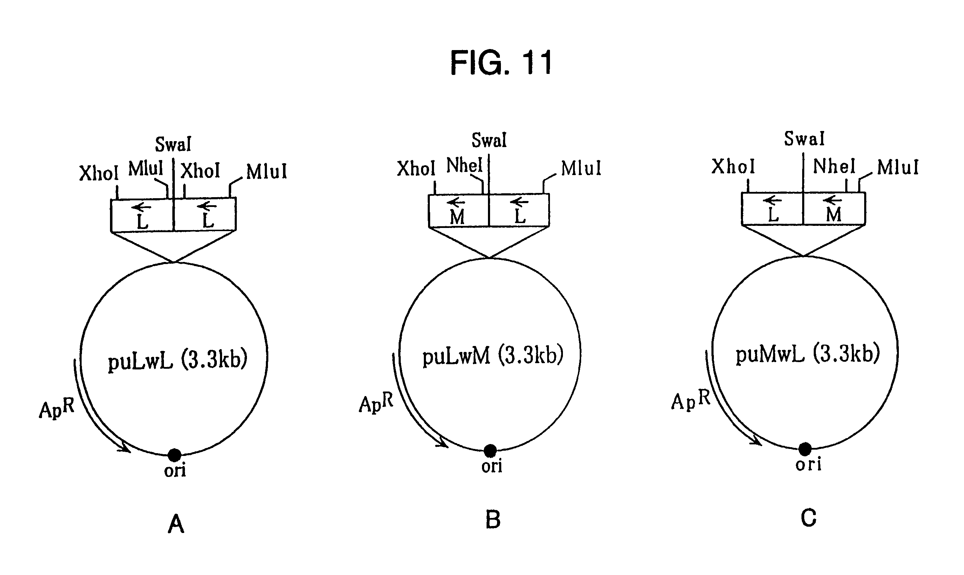

Plasmid puLL is a plasmid in which two wild-type loxP sites are inserted with the same direction into the restriction enzyme Ecl136II site of plasmid pUC119 (Kanegae et al., Nucleic Acids Res., 23: 3816-3821, 1995). The following operations were conducted in order to change the cloning site between the two loxP sites. The cloning site originally has a cleavage site specific for restriction enzyme NruI at each end. These sites are replaced with SwaI sites as follows:
puLL was digested with restriction enzyme NruI, and ligated with SwaI linker (5'-pGATTTAAATC-3', SEQ ID NO: 61), then again digested with NruI. Repeated digestion with NruI was performed to remove plasmid in which NruI site was regenerated without ligating SwaI linker. After transformation of E. coli with the reaction mixture, plasmid puLwL (3.3 kb, A in Fig. 11) having SwaI sites as a cloning site between two wild-type loxP sites was obtained.

### [2] Construction of plasmid (puLwM and puMwL) having cloning site (SwaI site) between wild-type loxP site and mutant loxP site

The following operations (A) and (B) were performed in order to construct two types of plasmids, in which one of two wild-type loxP sites in plasmid puLwL was replaced by mutant loxP site. Plasmid puLwM (B in Fig. 11) is a plasmid, in which wild-type loxP site in the left side of puLwL shown in the same figure is replaced by mutant loxP site, and plasmid puMwL (C in Fig. 11) is a plasmid, in which wild-type loxP site in the right side is replaced by mutant loxP site. In this experiment, as an example of a mutant loxP site, mutant sequence #2171 (SEQ ID NO: 26), in which bases at position 2 and 7 in the spacer region of wild-type loxP site were simultaneously mutated, was used. The mutant loxP site in the following examples is the same as this sequence.

### (A) Construction of puLwM

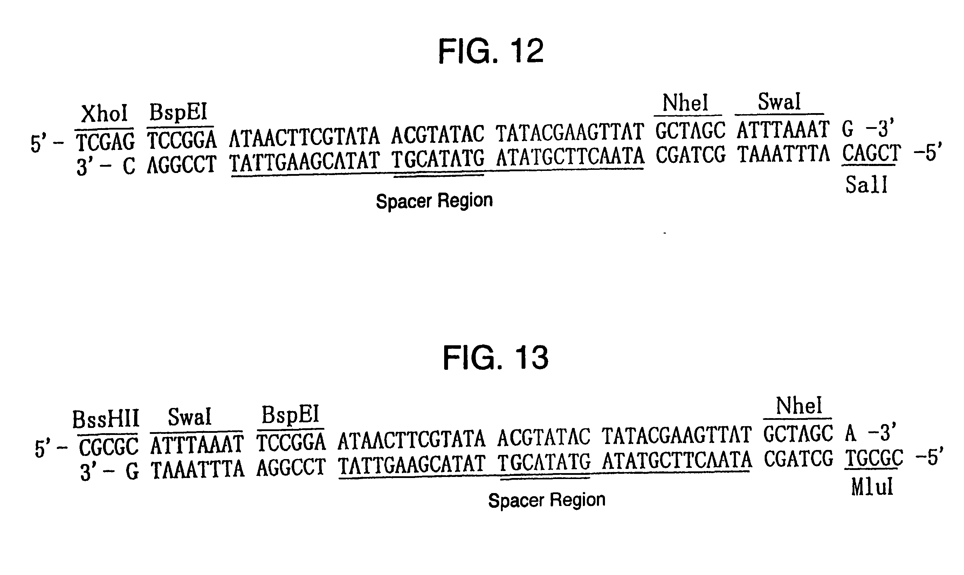

puLwL was digested with restriction enzyme XhoI, and ligated with synthetic DNA of 60 bp, containing the mutant loxP site (Fig. 12, upper strand, SEQ ID NO: 57 and lower strand SEQ ID NO: 58). After transformation of E. coli, plasmid puLwM (3.3 kb) bearing SwaI site between wild-type loxP site and mutant loxP site was obtained. The nucleotide sequence of the synthetic DNA part in plasmid puLwM was determined to confirm that wild-type loxP site and mutant loxP site were inserted as intended. Since the synthetic DNA used was designed to have the XhoI digested fragment in one end and SalI digested fragment in the other end, XhoI site in the wild-type loxP site of the plasmid puLwM was deleted.

### (B) Construction of puMwL

puLwL was digested with restriction enzyme MluI, and ligated with synthetic DNA of 60 bp containing the mutant loxP site (Fig. 13, upper strand SEQ ID NO: 59 and lower strand SEQ ID NO: 60). After transformation of E. coli, plasmid puMwL (3.3 kb) bearing SwaI site between wild-type loxP site and mutant loxP site was obtained. Further, as with plasmid puLwM, the nucleotide sequence in the synthetic DNA part was determined to confirm the exact insertion. Since the synthetic DNA used was designed to have the MluI digested fragment in one end and BssHII digested fragment in the other end, the MluI site in the wild-type loxP site of the plasmid puMwL was deleted.

### [3] Construction of plasmid (pCALL) inserted with two wild-type loxP sites into the cloning site of the CAG promoter

The following DNA were prepared in order to obtain a plasmid, to which two wild-type loxP sites were inserted between the promoter and poly(A) sequence in CAG promoter. The CAG promoter herein is disclosed in Japanese Patent Unexamined Publication No. Hei 3-168087 as a high expression vector.
(a) Plasmid pCAGw (Japanese Patent Unexamined Publication No. Hei 8-84589, page 10), in which the cloning site of plasmid pCAGGS (Niwa et al., Gene, 108: 193-200, 1991) containing CAG promoter was changed to replace EcoRI sites with SwaI sites, was digested with restriction enzyme SwaI to obtain a linear DNA fragment.
(b) Plasmid puLL (described in [1]) was doubly digested with BamHI and EcoRI, and both ends were blunted with Klenow enzyme, then subjected to agarose gel electrophoresis to obtain DNA fragment of approx. 100 bp, containing two wild-type loxP sites.

DNA fragments obtained from both (a) and (b) were ligated, and the ligation mixture was digested with restriction enzyme SwaI, then E. coli was transformed to obtain plasmid pCALL (4.9 kb).

### [4] Construction of plasmid (pCALwL), in which the cloning site between two loxP site of plasmid pCALL was changed to replace the NruI sites with SwaI sites.

The following operation was performed in order to replace the NruI sites with SwaI sites.

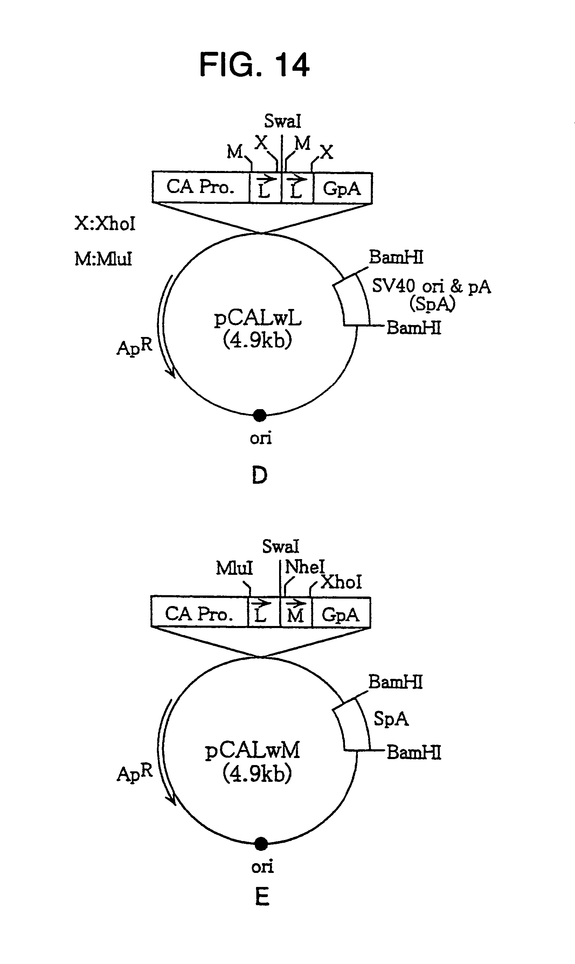

pCALL was digested with restriction enzyme NruI, and ligated with SwaI linker (5'-pGATTTAAATC-3', SEQ ID NO: 61), and again digested with restriction enzyme NruI. Reason for repeated digestion with NruI is same as in [1]. E. coli was transformed by reaction mixture to obtain plasmid pCALwL (4.9 kb, D in Fig. 14) having SwaI site as a cloning site between two wild-type loxP sites in the CAG promoter.

### [5] Construction of plasmid (pCALwM) inserting wild-type loxP site and mutant loxP site into the CAG promoter

The following operations were performed in order to obtain plasmid, in which one of the wild-type loxP sites in plasmid pCALwL is replaced by a mutant loxP site.
(a) Plasmid pCALwL was doubly digested with restriction enzymes MluI and XhoI, and DNA fragment of approx. 4.8 kb not containing wild-type loxP site was recovered after agarose gel electrophoresis.
(b) Plasmid puLwM was doubly digested with restriction enzymes MluI and XhoI, and DNA fragment of 100 bp containing both wild-type loxP site and mutant loxP site was recovered after agarose gel electrophoresis.

After ligation of both DNA fragments prepared in (a) and (b), E. coli was transformed to obtain plasmid pCALwM (4.9 kb, E in Fig. 14). pCALwM is a plasmid having structural component of promoter / wild-type loxP site / SwaI site (cloning site) / mutant loxP site / poly(A) sequence.

### [6] Construction of cosmid vector (pAxCALwM) for generation of recombinant adenovirus

The following operations were performed in order to construct a cosmid vector, in which the DNA from the promoter to the poly(A) sequence of plasmid pCALwM (prepared in [5]) was inserted. Construction of the cosmid vector was conducted according to known methods (Miyake et al. Proc. Natl. Acad. Sci., 93: 1320-1324, 1996 and Japanese Patent Unexamined Publication No. Hei 7-298877).
(a) Cassette cosmid pAxcw (Japanese Patent Unexamined Publication No. Hei 8-308585, page 15), to which almost full length of adenovirus type 5 DNA except for E1 and E3 gene was inserted, was digested with restriction enzyme SwaI.
(b) Plasmid pCALwM was simultaneously digested with restriction enzymes ApaLI, HindIII and HincII, then both ends were blunted with Klenow enzyme, and the reaction mixture was subjected to the agarose gel electrophoresis to recover the DNA fragment of approx. 2.4 kb.

After ligation of both DNA fragments prepared in (a) and (b), an aliquot of reaction mixture was packaged using lambda in vitro packaging kit (Gigapack XL, Stratagene Corp.). The reaction mixture was used to infect E. coli, and cassette cosmid pAxCALwM (44.9 kb) was obtained.

### (2) Construction of target plasmid for gene replacement

### [1] Construction of plasmid (puLZM) inserting lacZ gene between wild-type loxP site and mutant loxP site

Following operations were conducted in order to obtain plasmid inserting E. coli lacZ gene between wild-type loxP site and mutant loxP site in plasmid puLwM (described in (1)-[2]).
(a) Plasmid puLwM was digested with restriction enzyme SwaI, and linear DNA fragment was recovered after agarose gel electrophoresis.
(b) Plasmid pSRlacZ (Miyake et al., Proc. Natl. Acad. Sci., 93: 1320-1324, 1996) was doubly digested with restriction enzyme SalI and PstI, and the both ends were blunted with Klenow enzyme. A DNA fragment of approx. 3.1 kb containing lacZ gene was recovered after agarose gel electrophoresis.

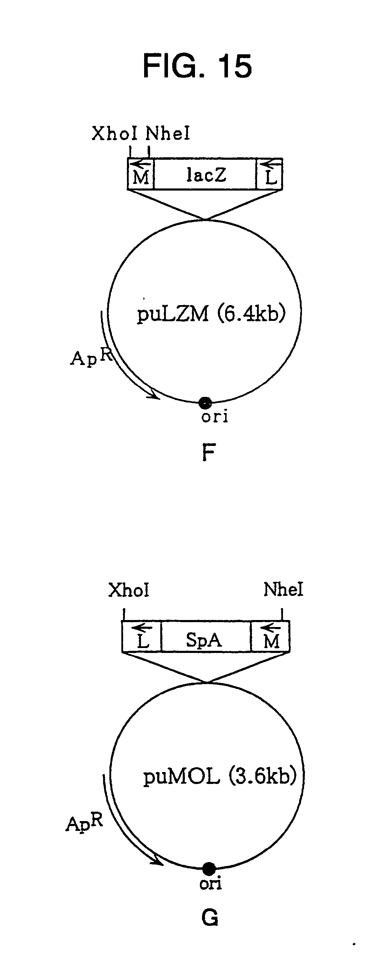

After ligation of both DNA fragments prepared in (a) and (b) and digestion with restriction enzyme SwaI, E. coli was transformed, and plasmid puLZM (6.4 kb, F in Fig. 15) was obtained.

### [2] Construction of plasmid (puMOL) inserting replication origin (ori) and poly(A) sequence of SV40 between wild-type loxP site and mutant loxP site

The following operations were conducted in order to obtain a plasmid comprising a replication origin (ori) and the poly(A) sequence of SV40 between the wild type loxP site and mutant loxP site in plasmid puMwL.
(a) Plasmid puMwL (described in (1)-[2]) was digested with restriction enzyme SwaI, and linear DNA fragment was recovered after agarose gel electrophoresis.
(b) Plasmid pCAGGS (described in (1)-[3]) was digested with restriction enzyme BamHI and both ends were blunted with Klenow enzyme. A DNA fragment of approx. 340 bp containing ori and the poly(A) sequence of SV40 was recovered after agarose gel electrophoresis.

After ligation of both DNA fragments prepared in (a) and (b) and digestion with restriction enzyme SwaI, E. coli was transformed, and plasmid puMOL (3.6 kb, G in Fig. 15) was obtained.

### [3] Construction of plasmid (puLZMOL) inserting wild-type loxP site / lacZ gene / mutant loxP site / ori and poly(A) sequence of SV40 / wild-type loxP site

The following operations were conducted in order to delete mutant loxP site from plasmid puLZM and insert the DNA fragment containing mutant loxP site /ori and poly(A) sequence of SV40 / wild-type loxP site.
(a) Plasmid puLZM was doubly digested with restriction enzymes NheI and XhoI, and the DNA fragment of approx. 6.4 kb containing wild-type loxP site and lacZ gene was recovered after agarose gel electrophoresis.
(b) Plasmid puMOL was doubly digested with restriction enzymes NheI and XhoI, and the DNA fragment of approx. 440 bp containing mutant loxP site / ori and poly(A) sequence of SV40 / wild-type loxP site was recovered after agarose gel electrophoresis.

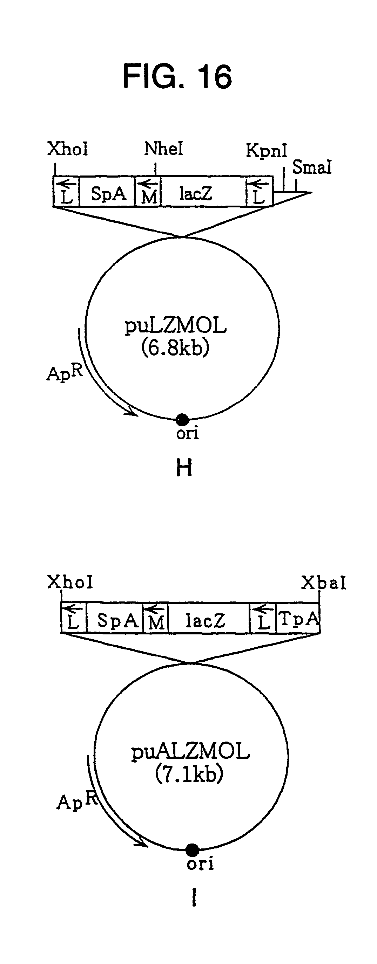

After ligation of both DNA fragments prepared in (a) and (b) and digestion with restriction enzyme SwaI, E. coli was transformed, and plasmid puLZMOL (6.8 kb, H in Fig. 16) was obtained.

### [4] Construction of plasmid (puALZMOL) inserting poly(A) sequence of thymidine kinase gene into 5'-upstream region of lacZ gene in plasmid puLZMOL

The following operations were conducted in order to construct a plasmid, in which the poly(A) sequence of the thymidine kinase (TK) gene of herpes simplex virus type 1 (HSV-1) is inserted into plasmid puLZMOL.
(a) A DNA fragment was obtained, in which plasmid puLZMOL was cleaved once at the 5'-upstream site of the wild-type loxP site located 5'-upstream region of the lacZ gene. To achieve this, puLZMOL was digested with restriction enzyme SmaI and then digested with restriction enzyme KpnI. A linear DNA fragment of approx. 6.8 kb was recovered after agarose gel electrophoresis.
(b) Plasmid pTK is a plasmid, in which BamHI digested fragment of approximately 3.6 kb containing TK gene of HSV-1, was inserted into BamHI site of pBR322 (M. Wigler et al., Cell, 14: 725-731, 1978). A poly(A) sequence of approximately 320 bp in the TK gene can be excised by digesting pTK with restriction enzymes SmaI and NcoI. The following operation can convert the ends of the excised DNA fragment to SmaI and KpnI sites. Plasmid pTK was digested with restriction enzyme NcoI and both ends were blunted with Klenow enzyme. After ligation with KpnI linker (5'-pGGGTACCC-3'), the reaction mixture was digested with restriction enzyme KpnI and then digested with restriction enzyme SmaI. The DNA fragment of approx. 320 bp containing poly(A) sequence of TK gene was recovered after agarose gel electrophoresis.

After ligation of both DNA fragments prepared in (a) and (b), E. coli was transformed, and plasmid puALZMOL (7.1 kb, I in Fig. 16) was obtained. puALZMOL is a plasmid having structural component of poly(A) sequence of TK gene / wild-type loxP site / lacZ gene /mutant loxP site / ori and poly(A) sequence of SV40 /wild-type loxP site. The poly(A) sequence of TK gene is inserted to prevent transcription of the lacZ gene from an unidentified adenovirus-derived promoter located upstream of the insertion site.

### [5] Construction of cosmid vector (pAxALZMOL) to generate a recombinant adenovirus

The following operation was conducted in order to construct the cosmid vector, in which the genes inserted in plasmid puALZMOL (poly(A) sequence of TK gene / wild-type loxP sire / lacz gene / mutant loxP site / ori and poly(A) sequence of SV40 / wild-type loxP site) are inserted.

Plasmid puALZMOL was simultaneously digested with restriction enzymes XbaI, XhoI and DraI, and both ends were blunted with Klenow enzyme, the reaction mixture was subjected to agarose gel electrophoresis, and the DNA fragment of approx. 4.5 kb was recovered. This DNA fragment and cassette cosmid pAxcw, digested with restriction enzyme SwaI (prepared in (1) - [6]) were ligated, and digested with restriction enzyme SwaI, and an aliquot of the reaction mixture was packaged using lambda in vitro packaging kit (Gigapack XL, Stratagene Inc.). The reaction mixture was used to infect to E. coli, and cassette cosmid pAxALZMOL (46.7 kb) was obtained.

### Example 6

### Generation of recombinant adenoviruses required for gene replacement

### (1) Generation of the target recombinant adenovirus (AxCALwM) for gene replacement

The following operation was conducted in order to generate a recombinant adenovirus, in which promoter / wild-type loxP site / SwaI site (cloning site) /mutant loxP site / poly(A) sequence, was inserted into the El gene deletion site in a replication-deficient adenovirus vector (deletion of E1 and E3 genes). Generation of recombinant adenovirus was performed according to a known method (Miyake et al., Proc. Natl. Acad. Sci., 93: 1320-1324, 1996 and Japanese Patent Unexamined Publication No. Hei 7-298877).

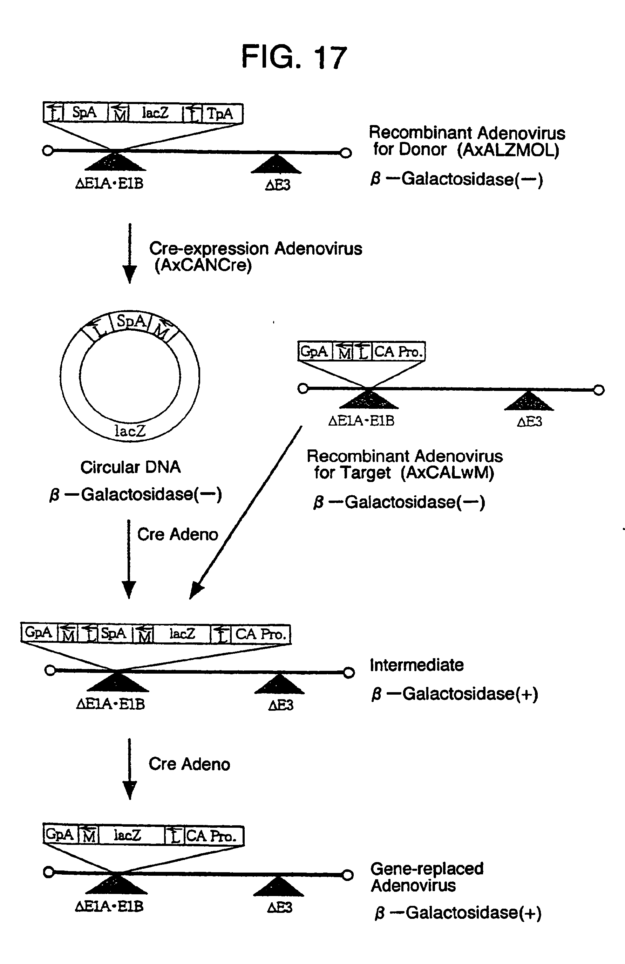

The viral DNA-terminal protein complex of Ad5-dlX having an E3 deletion (I. Saito et al., J. Virol., 54: 711-719, 1985), which is a strain derived from human adenovirus type 5, was digested with restriction enzyme EcoT22I. 293 cells were transfected with the viral DNA-terminal protein complex and cosmid vector pAxCALwM constructed in example 5-(1)-[6] by the calcium phosphate coprecipitation method. After the cloning and the selection of generated recombinant adenoviruses, the desired recombinant adenovirus AxCALwM (Fig. 17) was obtained. This recombinant adenovirus was subcultured and purified from 4th or 5th seed of virus stock with high titer by CsCl density-gradient centrifugation (Y. Kanegae et al., Jpn J. Med. Sci. Biol., 47: 157-166, 1994). The resulting purified adenovirus will be used for future experiments.

### (2) Generation of donor recombinant adenovirus (AxALZMOL) for gene replacement

The following operation was conducted in order to generate a recombinant adenovirus, in which poly(A) sequence of TK gene / wild-type loxP site / lacZ gene /mutant loxP site / ori and poly(A) sequence of SV40 /wild-type loxP site, were inserted into E1 gene deletion site in a replication-deficient adenovirus vector (E1 and E3 genes were deleted).

293 cells were transfected with the viral DNA-terminal protein complex (digested with EcoT22I as above) and cosmid vector pAxALZMOL (constructed in example 5-(2)-[5]) by the calcium phosphate coprecipitation method. The desired recombinant adenovirus AxALZMOL (Fig. 17) was obtained by means of the same method as in (1). This recombinant adenovirus was also purified from the 4th or 5th seed of virus stock by CsCl density-gradient centrifugation. The resulting purified adenovirus will be used for future experiments.

### Example 7

### Construction of cell lines for use to demonstrate replacement of a gene on a chromosome of a cell

### (1) Construction of a cell line for gene replacement expressing hygromycin B resistance gene

### [1] Construction of plasmid (pCALwMds) for reporter gene insertion having a cloning site between a wild-type loxP site and a mutant loxP site

Plasmid pCALwM (E in Fig. 14) prepared in example 5-(1)-[5] is a plasmid, in which wild-type loxP site / cloning site (SwaI site) / mutant loxP site is inserted into an EcoRI site (a cloning site in the CAG promoter of plasmid pCAGGS, described in example 5-(1)-[3]). The following operation was conducted in order to remove both ori and poly(A) sequence of SV40 from the pCALwM.

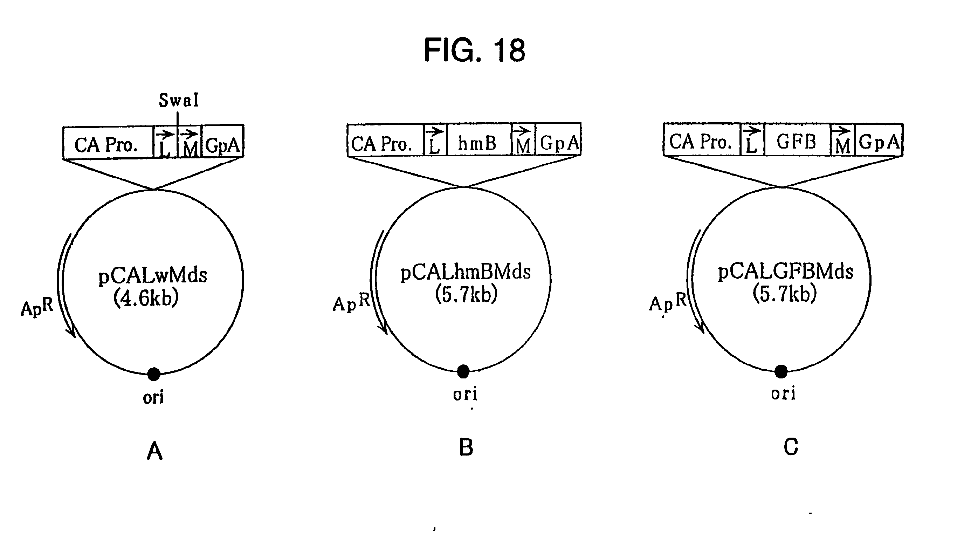

pCALwM was digested with BamHI to remove ori and poly(A) sequence of SV40, then after self-ligation, E. coli was transformed, and plasmid pCALwMds (4.6 kb, A in Fig. 18) was obtained:

### [2] Construction of plasmid (pCALhmBMds) inserted with hygromycin B resistance gene

The following DNA was prepared in order to construct a plasmid, in which hygromycin B resistance gene was inserted into the SwaI site of plasmid pCALwMds.
(a) a DNA fragment obtained by digestion of plasmid pCALwMds with restriction enzyme SwaI.
(b) a DNA fragment of approx. 1.1 kb, containing hygromycin B resistant gene, recovered from agarose gel electrophoresis, after double digestion of plasmid pCHD2L (Ikeda et al., Gene, 71: 19-27, 1988) with restriction enzymes SmaI and DraI.

After ligation of both DNA fragments of (a) and (b), E. coli was transformed, and plasmid pCALhmBMds (5.7 kb, B in Fig. 18) was obtained. pCALhmBMds is a plasmid, in which hygromycin B resistance gene is inserted between wild-type loxP site and mutant loxP site in the CAG promoter.

### [3] construction or transformed cell line expressing hygromycin B resistance gene

The following operation was conducted in order to obtain a transformed cell line having one copy of foreign DNA consisting of promoter / wild-type loxP site / hygromycin B resistance gene / mutant loxP site /poly(A) sequence on chromosome of cells. Transformation of cells was performed using the calcium phosphate DNA coprecipitation method of Chen-Okayama method.
(i) 20µg of plasmid pCALhmBMds constructed in [2] was dissolved in final concentration of 250 mM calcium chloride solution (volume 100µl), and 100µl of 2 x BBS solution (50 mM N,N-bis[2-hydroxyethyl]-2-aminoethane sulfonic acid / 280 mM NaCl / 1.5 mM Na₂HPO₄ (pH 6.95) were added. This mixture was added to CV-1 cells cultured in the DMEM medium supplemented with 5% FCS in 6cm dish, and cultured at 35°C for about 24 hours in 3% CO₂ atmosphere.
(ii) After removal of culture medium, cells were washed with PBS(-), and were added to DMEM medium supplemented with 10% FCS, and cultured at 37°C for overnight in 5% CO₂ atmosphere.
(iii) The cells were harvested from 6cm dish and inoculated to 96 well microplates and further cultured for overnight. Medium containing hygromycin B was added to the cells to final concentration of 0.4 mg/ml, cells were further continued for cultivation, and medium containing hygromycin B was exchanged on 3 to 4 days intervals.
(iv) Surviving cells after about 3 weeks (hygromycin B resistant cell lines) were cloned and expanded, and genomic DNA was extracted from the cell lines. To obtain transformed cell lines, in which only single copy of the objective gene was inserted on a cell chromosome, the genomic DNAs were digested with restriction enzyme PvuII (there is no PvuII recognition site in the plasmid pCALhmBMds used for transformation), and digested DNAs were analyzed by Southern blotting using the full length of plasmid pCALhmBMd as a probe. As a result, 18 clones of desired cell lines were obtained. Insertion of a single copy of the objective gene was also confirmed by Southern blotting analysis of the genomic DNAs of cells digested with restriction enzyme EcoRI, for which only one recognition site exists on plasmid pCALhmBMd.

### (2) Construction of cell lines for gene replacement expressing bleomycin resistant gene

### [1] Construction of plasmid (pCALGFBMds) inserted with bleomycin resistant gene

The following operation was conducted in order to construct a plasmid by inserting the bleomycin resistance gene into the SwaI site of plasmid pCALwMds.

Plasmid pTracer-CMV (Invitrogene Inc.) was digested with restriction enzyme PmaCI, and subjected to agarose gel electrophoresis, and then a DNA fragment of approx. 1.1 kb containing a fusion gene of green fluorescent protein (GFP) and the bleomycin resistance gene was recovered. The said DNA fragment was ligated with SwaI digested plasmid pCALwMds (prepared in (1)-[2]). The reaction mixture was digested with restriction enzyme SwaI and used to transfect E. coli. Plasmid pCALGFBMds (5.7 kb, C in Fig. 18) was obtained. Plasmid pCALGFBMds is a plasmid, in which a fusion gene of GFP and the bleomycin resistance gene is inserted between the wild-type loxP site and the mutant loxP site in the CAG promoter.

### [2] Construction of a transformed cell line expressing bleomycin resistance gene

The following operation was conducted in order to obtain a transformed cell line having single copy of foreign DNA, consisting of promoter / wild-type loxP site / fusion gene of GFP and the bleomycin resistance gene /mutant loxP site / poly(A) sequence.

Basically, the method is the same as in the construction of hygromycin B resistant cell lines shown in (1)-[3]. The only different procedures are explained as follows. The plasmid pCALGFBMds constructed in [2], was used for transformation of CV-1 cells. Bleomycin (final concentration 0.4 mg/ml) was used for drug selection of transformed cells. Full length DNA of pCALGFBMds was used as a probe for Southern blotting for selection of transformants inserted with only single copy of the objective gene.

The expression level of GFP was measured to classify the expression levels of the inserted gene in the obtained bleomycin resistant. Expression level of GFP was measured by detecting fluorescence at 507 nm by excitation at 478 nm. As a result, cells were classified into: cell lines with high expression of GFP (C18 and C35), cell lines with medium expression (C29 and C38) and cell lines with low expression (C19 and C30)(Numerals show number of cell line).

### Example 8

### Replacement of gene on adenovirus genome

The following experiments were conducted in order to prove that the lacZ gene in the plasmid DNA (circular DNA) could be used to replace a gene in the adenovirus genome by combination of a wild-type loxP site and a mutant loxP site. The principle of the experiment is as follows (Fig. 17). Poly(A) sequence of TK gene / wild-type loxP site / lacZ gene / mutant loxP site / ori and poly(A) sequence of SV40 / wild-type loxP site are inserted into donor recombinant adenovirus AxALZMOL generated in example 6. When the cultured cells were doubly infected with this virus and Cre expressing recombinant adenovirus AxCANCre (shown in example 1), specific recombination occurred between two wild-type loxP sites. Adenovirus (a) having poly(A) sequence of TK gene / wild-type loxP site and circular DNA (b) having wild-type loxP site / lacZ gene / mutant loxP site / ori and poly(A) sequence of SV40 were generated. Since the efficiency of this reaction using recombinant adenovirus inserted with two wild-type loxP sites and Cre expressing recombinant adenovirus is very high (Kanegae et al., Nucleic Acids Res., 23: 3816-3821, 1995), it is expected to generate adenovirus (a) and circular DNA (b) in almost 100% of cultured cells. Though there is a promoter of SV40 ori in the circular DNA (b), lacZ gene can not express, because the transcription of lacZ is blocked by the poly(A) sequence of SV40, which is located adjacently downstream of the promoter. When cells, in which the circular DNA (b) was generated, are further infected with target recombinant adenovirus AxCALwM generated in example 6, recombination between the circular DNA (b) and AxCALwM occurs. Adenovirus (c) having structure of promoter / wild-type loxP site / lacZ gene / mutant loxP sequence / poly(A) sequence is generated through intermediates containing two wild-type loxP sites and two mutant loxP sites. In adenovirus (c), lacZ gene is expressed, and β-galactosidase encoded in lacZ gene is produced. Accordingly cells are stained blue by staining treatment as follows.

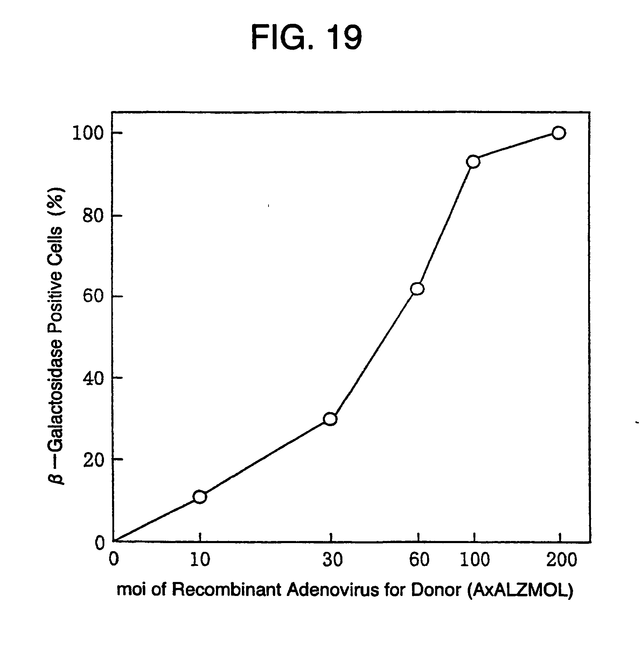

In the actual experiment, cultured cells (CV-1 cells or COS-1 cells) were simultaneously infected with three types of viruses including donor recombinant adenovirus AxALZMOL, Cre expressing recombinant adenovirus AxCANCre and target recombinant adenovirus AxCALwM. Multiplicity of infection (moi) or each virus is as follows. Recombinant adenovirus for target: moi = 9, Cre expressing recombinant adenovirus: moi = 5 or 15 and recombinant adenovirus for donor: moi = 10, 30, 60, 100 and 200. Cells were infected with above viruses for 1 hour, then medium was added and the cells cultured. Three days later, cultured medium was removed and cells were washed with PBS(-) at their surface, and cells were fixed with 0.25% glutaraldehyde solution at 4°C for 10 minutes, then again washed with PBS(-). X-Gal staining solution [5mM potassium ferricyanide / 5 mM potassium ferrocyanide / 2 mM magnesium chloride / 1 mg/ml X-Gal (5-bromo-4-chloro-3-indolyl-b-D-galactoside) / PBS(-)] was added and stained for overnight. Results are shown in Fig. 19. In the case, CV-1 cells were infected with Cre expressing recombinant adenovirus at moi 15. About 60% of cells infected with donor recombinant adenovirus at moi 60, and about 90% of cells infected at moi 100, were stained. Although not shown in Fig. 19, no blue stained cells were found when cells were infected with only donor recombinant adenovirus or doubly infected with donor recombinant adenovirus and Cre expressing recombinant adenovirus. Further, in a case where cells were infected with Cre expressing recombinant adenovirus at moi 5, and the same experiment using COS-1 cells was conducted, almost same result as in Fig. 19 was obtained.

The above results indicate that the lacZ gene in the genome of the donor recombinant adenovirus was inserted into the genome of the target recombinant adenovirus. The inserted lacZ gene was connected directly with a promoter, β-galactosidase was expressed and cells were stained with blue. The result clearly proves that, via the formation of circular DNA, the lacZ gene between the wild-type loxP site and the mutant loxP site in the genome of the donor recombinant adenovirus, replaces with very high efficiency the sequence between the wild-type loxP site and the mutant loxP site in the genome of the target recombinant adenovirus.

### Example 9

### Replacement of a gene on a chromosome of a cell

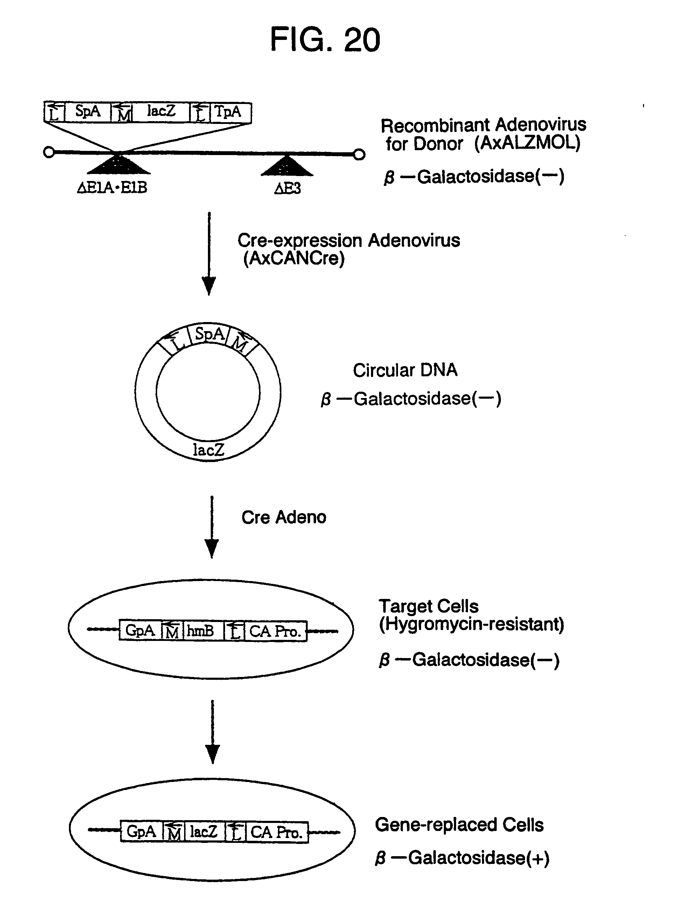

In example 8, it was shown that a gene in circular DNA could be inserted in the genome of adenovirus. In order to prove that a gene can also be inserted into a chromosome of a cell, the following experiment was conducted. The principle is same as in example 8, but instead of a target recombinant adenovirus, transformed cell lines (target cell)as constructed in example 7 (in which a single copy of DNA consisting of promoter / wild-type loxP site /hygromycin B resistance gene / mutant loxP site /poly(A) sequence are inserted into a chromosome) were used (Fig. 20).

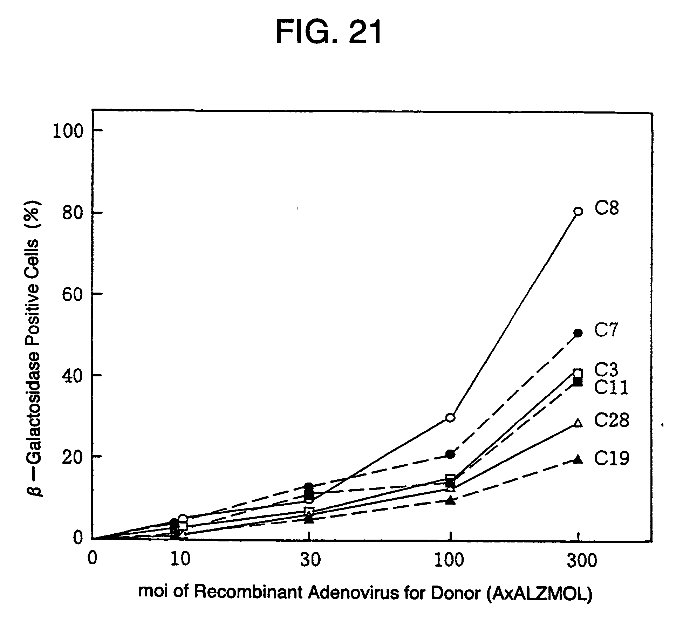

Each of any six target cells with hygromycin B resistance constructed in example 7 (C3, C7, C8, C11, C19 and C20) were doubly infected with Cre expressing recombinant adenovirus AxCANCre at moi 5 and donor recombinant adenovirus AxALZMOL at mois of 10, 30, 100 and 300, respectively, for 1 hour, then medium was added and the cells cultured. Three days later, the same operation as shown in example 8 was performed and cells expressing β-galactosidase were stained. Results are shown in Fig. 21. Although the frequency of expression of β-galactosidase was different depending on the cell lines, when cells were infected with donor recombinant adenovirus at moi 100, cells from 10% (C19) to 30% (C8) were stained with blue. When cells were infected with donor recombinant adenovirus at moi 300, the ratio of cells stained with blue was further increased. However toxicity to cells due to the large amount of adenovirus particles was observed. Although the data is not shown in figure 20, when cells were infected with only donor recombinant adenovirus at moi 100, without Cre expressing recombinant adenovirus, there were no cells stained with blue at all.

The above results clearly prove that, via the formation of circular DNA, the lacZ gene between the wild-type loxP site and the mutant loxP site in the genome of the donor recombinant adenovirus replaces with high efficiency the sequence between the wild-type loxP site and the mutant loxP site in the genome of the target cells.

### Free Text of Sequence Listing

SEQ ID NO: 1: wild-type loxP site;
SEQ ID NO: 2 - 54: mutant loxP site;
SEQ ID NO: 55: sequence of sense strand designed for containing wild-type loxP site and restriction enzyme recognizing site;
SEQ ID NO: 56: sequence of antisense strand designed for containing wild-type loxP site and restriction enzyme recognizing site;
SEQ ID NO: 57: sequence of sense strand designed for containing mutant loxP site and restriction enzyme recognizing site;
SEQ ID NO: 58: sequence of antisense strand designed for containing mutant loxP site and restriction enzyme recognizing site;
SEQ ID NO: 59: site of sense strand designed for containing mutant loxP site and restriction enzyme recognizing site;
SEQ ID NO: 60: sequence of antisense strand designed for containing mutant loxP site and restriction enzyme recognizing site; and
SEQ ID NO: 61: SwaI linker.

## Claims

1. A mutant loxP site having the following properties:
(a) a nucleotide sequence of the following formula derived from a wild-type E. coli P1 phage loxP site, wherein at least one of the bases consisting of the second (T), third (G), fourth (T) and fifth (A) bases, and at least one of the bases consisting of the sixth (T) and seventh (G) bases within the 8 bases in the central part (spacer region) of the sequence are substituted by different bases, and bases outside the spacer region are optionally substituted;
| | 12345678 | |
|---|---|---|
| 5'ATAACTTCGTATA | ATGTATGC | TATACGAAGTTAT-3' |
| | | |
| | Spacer Region | |
(b) a specific DNA recombination between said mutant loxP site and the wild-type loxP site cannot occur in the presence of recombinase Cre; and
(c) a specific DNA recombination between the mutant loxP sites having identical nucleotide sequences can occur in the presence of recombinase Cre.

2. A mutant loxP site having the following properties:
(a) a nucleotide sequence of the following formula derived from a wild-type E. coli P1 phage loxP site, wherein a base selected from the group consisting of second (T), third (G) and fourth (T) bases of the spacer region is substituted by a different base, and bases outside the spacer region are optionally substituted by any base;
| | 12345678 | |
|---|---|---|
| 5'-ATAACTTCGTATA | ATGTATGC | TATACGAAGTTAT-3' |
| | Spacer Region | |
(b) a specific DNA recombination between said mutant loxP site and the wild-type loxP site cannot occur in the presence of recombinase Cre; and
(c) a specific DNA recombination between the mutant loxP sites having identical nucleotide sequences can occur in the presence of recombinase Cre.

3. The mutant loxP site according to claim 1 or 2, wherein specific DNA recombination between the mutant loxP site and another mutant loxP site having a different nucleotide sequence cannot occur in the presence of recombinase Cre.

4. The mutant loxP site according to claim 1, having the sequence of SEQ ID NO: 26, SEQ ID NO: 29, SEQ ID NO: 30, SEQ ID NO: 35, SEQ ID NO: 39, SEQ ID NO: 42, or SEQ ID NO: 49.

5. A DNA comprising the mutant loxP site according to any one of claims 1 to 4.

6. A DNA comprising at least one wild-type loxP site and at least one mutant loxP site according to any one of claims 1 to 4.

7. The DNA according to claim 6 wherein a desired gene is inserted between the wild-type loxP site and the mutant loxP site.

8. A DNA comprising at least two mutant loxP sites according to claim 1 or 2, wherein the two sites have different nucleotide sequences and wherein specific DNA recombination between the two sites cannot occur in the presence of a recombinase Cre.

9. The DNA according to claim 8 wherein a desired gene is inserted between two mutant loxP sites having different nucleotide sequences to each other.

10. A non-human cell or a human cell cultured ex vivo which is transformed by DNA according to any one of claims 6 to 9, provided that the luman cell is not a germ cell and not an embryonic cell.

11. A method for replacing a gene A by a different gene B comprising reacting DNA (a) and DNA (b) in the presence of recombinase Cre: wherein DNA (a) is a DNA comprising a wild-type loxP site, gene A and a mutant loxP site according to claim 1 or 2, in this order; and DNA (b) is a circular DNA comprising a wild-type loxP site, a gene B and the same mutant loxP sequence as DNA (a), in this order; to obtain DNA (c) in which gene A is replaced by gene B in DNA (a).

12. A method for replacing a gene A by a different gene B comprising reacting DNA (a) and DNA (b) in the presence of recombinase Cre: wherein DNA (a) is a DNA comprising two mutant loxP sites having different nucleotide sequences wherein specific DNA recombination between the two sites cannot occur in the present of a recombinase Cre (mutant loxP site 1 and mutant loxP site 2) and gene A, arranged in the order of mutant loxP site 1 / gene A / mutant loxP site 2; DNA (b) is a circular DNA comprising the mutant loxP site 1, the gene B and the mutant loxP site 2, in this order; to obtain DNA (c) in which gene A is replaced by gene B in DNA (a).

13. The method according to claim 11 or 12 wherein the gene B is not a functional gene.

14. The method according to claim 11 or 12 wherein the gene A is not a functional gene.

15. The method according to any one of claims 11 to 14 wherein DNA (a) is chromosomal DNA of a cell and DNA (b) is a plasmid DNA or DNA of a double-stranded circular DNA virus.

16. The method according to any one of claims 11 to 14 wherein DNA (a) is chromosomal DNA of a cell and DNA (b) has properties to be converted intracellularly to double-stranded circular DNA.

17. The method according to any one of claims 11 to 14 wherein DNA (a) is chromosomal DNA of a double-stranded DNA virus and DNA (b) is a plasmid DNA or DNA of a double-stranded circular DNA virus.

18. The method according to any one of claims 11 to 14 wherein DNA (a) is chromosomal DNA of a double-stranded DNA virus and DNA (b) has properties to be converted intracellularly to double-stranded circular DNA.

19. The method according to claim 17 or 18 wherein the double-stranded DNA virus is adenovirus.

20. A non-human transgenic animal having DNA according to any one of claims 6 to 9 on the chromosome.

21. A pharmaceutical product comprising DNA according to any one of claims 6 to 9.

## Patentansprüche

1. Mutierte loxP-Stelle, die die folgenden Eigenschaften hat:
(a) eine Nucleotidsequenz der folgenden Formel, die von einer Wildtyp-E.coli-P1 Phagen-loxP-Stelle abgeleitet ist, wobei mindestens eine der Basen bestehend aus den zweiten (T), dritten (G), vierten (T) und fünften (A) Basen und mindestens eine der Basen bestehend aus den sechsten (T) und siebten (G) Basen innerhalb der 8 Basen im zentralen Teil (Spacer-Region) der Sequenz mit unterschiedlichen Basen substituiert sind, und Basen außerhalb der Spacer-Region gegebenenfalls substituiert sind;
| | 12345678 | |
|---|---|---|
| 5'ATAACTTCGTATA | ATGTATGC | TATACGAAGTTAT-3' |
| | Spacer-Region | |
(b) eine spezifische DNA-Rekombination zwischen der mutierten loxP-Stelle und der Wildtyp-loxP-Stelle kann nicht in Gegenwart der Rekombinase Cre stattfinden; und
(c) eine spezifische DNA-Rekombination zwischen den mutierten loxP-Stellen, die identische Nucleotidsequenzen haben, kann in Gegenwart der Rekombinase Cre stattfinden.

2. Mutierte loxP-Stelle, die die folgenden Eigenschaften hat:
(a) eine Nucleotidsequenz der folgenden Formel, die von einer Wildtyp-E.coli-P1 Phagen-loxP-Stelle abgeleitet ist, wobei eine Base ausgewählt aus der Gruppe bestehend aus zweiten (T), dritten (G) und vierten (T) Basen der Spacer-Region mit einer unterschiedlichen Base ersetzt ist, und Basen außerhalb der Spacer-Region gegebenenfalls durch eine beliebige Base ersetzt sind;
| | 12345678 | |
|---|---|---|
| 5'ATAACTTCGTATA | ATGTATGC | TATACGAAGTTAT-3' |
| | Spacer-Region | |
(b) eine spezifische DNA-Rekombination zwischen der mutierten loxP-Stelle und der Wildtyp-loxP-Stelle kann nicht in Gegenwart der Rekombinase Cre stattfinden; und
(c) eine spezifische DNA-Rekombination zwischen den mutierten loxP-Stellen, die identische Nucleotidsequenzen haben, kann in Gegenwart der Rekombinase Cre stattfinden.

3. Mutierte loxP-Stelle nach Anspruch 1 oder 2, wobei eine spezifische DNA-Rekombination zwischen der mutierten loxP-Stelle und einer weiteren mutierten loxP-Stelle, die eine unterschiedliche Nucleotidsequenz hat, nicht in Gegenwart der Rekombinase Cre stattfinden kann.

4. Mutierte loxP-Stelle nach Anspruch 1, die die Sequenz der SEQ ID NO: 26, SEQ ID NO: 29, SEQ ID NO: 30, SEQ ID NO: 35, SEQ ID NO: 39, SEQ ID NO: 42 oder SEQ ID NO: 49 hat.

5. DNA, umfassend die mutierte loxP-Stelle nach einem der Ansprüche 1 bis 4.

6. DNA, umfassend mindestens eine Wildtyp-loxP-Stelle und mindestens eine mutierte loxP-Stelle nach einem der Ansprüche 1 bis 4.

7. DNA nach Anspruch 6, wobei ein gewünschtes Gen zwischen der Wildtyp-loxP-Stelle und der mutierten loxP-Stelle eingefügt ist.

8. DNA, umfassend mindestens zwei mutierte loxP-Stellen nach Anspruch 1 oder 2, wobei die zwei Stellen unterschiedliche Nucleotidsequenzen haben und wobei eine spezifische DNA-Rekombination zwischen den zwei Stellen nicht in der Gegenwart einer Rekombinase Cre stattfinden kann.

9. DNA nach Anspruch 8, wobei ein gewünschtes Gen zwischen zwei mutierten loxP-Stellen eingefügt ist, die voneinander verschiedene Nucleotidsequenzen haben.

10. Nicht-menschliche Zelle oder menschliche Zelle, die ex vivo gezüchtet ist, die mit einer DNA nach einem der Ansprüche 6 bis 9 transformiert ist, mit der Maßgabe, dass die menschliche Zelle keine Keimzelle und keine embryonale Zelle ist.

11. Verfahren zum Ersetzen eines Gens A mit einem unterschiedlichen Gen B, umfassend das Umsetzen von DNA (a) und DNA (b) in Gegenwart der Rekombinase Cre:
wobei DNA (a) eine DNA ist, die eine Wildtyp-loxP-Stelle, Gen A und eine mutierte loxP-Stelle nach Anspruch 1 oder 2 umfasst, in dieser Reihenfolge; und DNA (b) eine ringförmige DNA ist, umfassend eine Wildtyp-loxP-Stelle, ein Gen B und die gleiche mutierte loxP-Sequenz wie DNA (a), in dieser Reihenfolge; um DNA (c) zu erhalten, in der Gen A durch Gen B in DNA (a) ersetzt ist.

12. Verfahren zum Ersetzen eines Gens A mit einem unterschiedlichen Gen B, umfassend das Umsetzen von DNA (a) und DNA (b) in Gegenwart der Rekombinase Cre:
wobei DNA (a) eine DNA ist, umfassend zwei mutierte loxP-Stellen mit unterschiedlichen Nucleotidsequenzen, wobei eine spezifische DNA-Rekombination zwischen den zwei Stellen nicht in Gegenwart einer Rekombinase Cre stattfinden kann (mutierte loxP-Stelle 1 und mutierte loxP-Stelle 2), und Gen A, angeordnet in der Reihenfolge mutierte loxP-Stelle 1 / Gen A / mutierte loxP-Stelle 2;
DNA (b) eine ringförmige DNA ist, umfassend die mutierte loxP-Stelle 1, das Gen B und die mutierte loxP-Stelle 2, in dieser Reihenfolge;
um DNA (c) zu erhalten, in der Gen A mit Gen B in DNA (a) ersetzt ist.

13. Verfahren nach Anspruch 11 oder 12, wobei das Gen B kein funktionelles Gen ist.

14. Verfahren nach Anspruch 11 oder 12, wobei das Gen A kein funktionelles Gen ist.

15. Verfahren nach einem der Ansprüche 11 bis 14, wobei DNA (a) chromosomale DNA einer Zelle und DNA (b) eine Plasmid-DNA oder DNA eines doppelsträngigen ringförmigen DNA-Virus ist.

16. Verfahren nach einem der Ansprüche 11 bis 14, wobei DNA (a) chromosomale DNA einer Zelle ist und DNA (b) die Eigenschaften hat, intrazellulär in doppelsträngige ringförmige DNA umgewandelt zu werden.

17. Verfahren nach einem der Ansprüche 11 bis 14, wobei DNA (a) chromosomale DNA eines doppelsträngigen DNA-Virus und DNA (b) eine Plasmid-DNA oder DNA eines doppelsträngigen ringförmigen DNA-Virus ist.

18. Verfahren nach einem der Ansprüche 11 bis 14, wobei DNA (a) chromosomale DNA eines doppelsträngigen DNA-Virus ist und DNA (b) die Eigenschaften hat, intrazellulär in doppelsträngige ringförmige DNA umgewandelt zu werden.

19. Verfahren nach Anspruch 17 oder 18, wobei das doppelsträngige DNA-Virus Adenovirus ist.

20. Nicht-menschliches transgenes Tier, das DNA nach einem der Ansprüche 6 bis 9 auf dem Chromosom hat.

21. Pharmazeutisches Produkt, umfassend DNA nach einem der Ansprüche 6 bis 9.

## Revendications

1. Site loxP mutant ayant les propriétés suivantes :
(a) une séquence de nucléotides de formule suivante dérivée d'un site loxP de phage P1 de E. coli de type sauvage, dans laquelle au moins l'une des bases comprenant les deuxième (T), troisième (G), quatrième (T) et cinquième (A) bases et au moins l'une des bases comprenant les sixième (T) et septième (G) bases dans les 8 bases de la partie centrale (région d'espacement) de la séquence sont substituées par différentes bases et les bases se trouvant en dehors de la région d'espacement sont éventuellement substituées ;
| | 12345678 | |
|---|---|---|
| 5'ATAACTTCGTATA | ATGTATGC | TATACGAAGTTAT-3' |
| | région d'espacement | |
(b) une recombinaison d'ADN spécifique entre ledit site loxP mutant et le site loxP de type sauvage ne peut pas se produire en présence de la CRE recombinase ; et
(c) une recombinaison d'ADN spécifique entre les sites loxP mutants ayant des séquences de nucléotides identiques peut se produire en présence de la CRE recombinase.

2. Site loxP mutant ayant les propriétés suivantes :
(a) une séquence de nucléotides de formule suivante dérivée d'un site loxP de phage P1 de E. coli de type sauvage, dans laquelle une base choisie dans le groupe comprenant les deuxième (T), troisième (G) et quatrième (T) bases de la région d'espacement est substituée par une base différente, et des bases en dehors de la région d'espacement sont éventuellement substituées par une base quelconque :
| | 12345678 | |
|---|---|---|
| 5'-ATAACTTCGTATA | ATGTATGC | TATACGAAGTTAT-3' |
| | région d'espacement | |
(b) une recombinaison d'ADN spécifique entre ledit site loxP mutant et le site loxP de type sauvage ne peut pas se produire en présence de la CRE recombinase ; et
(c) une recombinaison d'ADN spécifique entre les sites loxP mutants ayant des séquences de nucléotides identiques peut se produire en présence de la CRE recombinase.

3. Site loxP mutant selon la revendication 1 ou 2, dans lequel la recombinaison d'ADN spécifique entre le site loxP mutant et un autre site loxP mutant ayant une séquence de nucléotides différente ne peut pas se produire en présence de la CRE recombinase.

4. Site loxP mutant selon la revendication 1, ayant la séquence de SEQ ID N° : 26, SEQ ID N° : 29, SEQ ID N° : 30, SEQ ID N° : 35, SEQ ID N° : 39, SEQ ID N° : 42 ou SEQ ID N° : 49.

5. ADN comprenant le site loxP mutant selon l'une quelconque des revendications 1 à 4.

6. ADN comprenant au moins un site loxP de type sauvage et au moins un site loxP mutant selon l'une quelconque des revendications 1 à 4.

7. ADN selon la revendication 6, dans lequel un gène souhaité est inséré entre le site loxP de type sauvage et le site loxP mutant.

8. ADN comprenant au moins deux sites loxP mutants selon la revendication 1 ou 2, dans lequel les deux sites ont différentes séquences de nucléotides et dans lequel la recombinaison d'ADN spécifique entre les deux sites ne peut pas se produire en présence d'une CRE recombinase.

9. ADN selon la revendication 8, dans lequel un gène souhaité est inséré entre deux sites loxP mutants ayant réciproquement différentes séquences de nucléotides.

10. Cellule non humaine ou cellule humaine cultivée *ex vivo* qui est transformée par l'ADN selon l'une quelconque des revendications 6 à 9, à condition que la cellule humaine ne soit pas une cellule germinale ni une cellule embryonnaire.

11. Procédé de remplacement d'un gène A par un gène B différent comprenant la réaction de l'ADN (a) et de l'ADN (b) en présence de la CRE recombinase, dans lequel l'ADN (a) est un ADN comprenant un site loxP de type sauvage, le gène A et un site loxP mutant selon la revendication 1 ou 2, dans cet ordre ; et l'ADN (b) est un ADN circulaire comprenant un site loxP de type sauvage, un gène B et la même séquence loxP mutante que l'ADN (a), dans cet ordre ; pour obtenir un ADN (c) dans lequel le gène A est remplacé par le gène B dans l'ADN (a).

12. Procédé de remplacement d'un gène A par un gène B différent, comprenant la réaction de l'ADN (a) et de l'ADN (b) en présence de CRE recombinase, dans lequel l'ADN (a) est un ADN comprenant deux sites loxP mutants ayant des séquences de nucléotides différentes, la recombinaison d'ADN spécifique entre les deux sites ne pouvant se produire en présence d'une CRE recombinase (site loxP mutant 1 et site loxP mutant 2) et un gène A, disposés dans l'ordre suivant : site loxP mutant 1 / gène A / site loxP mutant 2 ; l'ADN (b) étant un ADN circulaire comprenant le site loxP mutant 1, le gène B et le site loxP mutant 2, dans cet ordre ; pour obtenir un ADN (c) dans lequel le gène A est remplacé par le gène B dans l'ADN (a).

13. Procédé selon la revendication 11 ou 12, dans lequel le gène B n'est pas un gène fonctionnel.

14. Procédé selon la revendication 11 ou 12, dans lequel le gène A n'est pas un gène fonctionnel.

15. Procédé selon l'une quelconque des revendications 11 à 14, dans lequel l'ADN (a) est l'ADN chromosomique d'une cellule et l'ADN (b) est un ADN de plasmide ou un ADN d'un virus à ADN circulaire à double brin.

16. Procédé selon l'une quelconque des revendications 11 à 14, dans lequel l'ADN (a) est l'ADN chromosomique d'une cellule et l'ADN (b) a des propriétés lui permettant d'être converti au niveau intracellulaire en ADN circulaire à double brin.

17. Procédé selon l'une quelconque des revendications 11 à 14, dans lequel l'ADN (a) est l'ADN chromosomique d'un virus à ADN à double brin et l'ADN (b) est un ADN de plasmide ou un ADN d'un virus à ADN circulaire à double brin.

18. Procédé selon l'une quelconque des revendications 11 à 14, dans lequel l'ADN (a) est un ADN chromosomique d'un virus à ADN à double brin et l'ADN (b) a des propriétés lui permettant d'être converti au niveau intracellulaire en ADN circulaire à double brin.

19. Procédé selon la revendication 17 ou 18, dans lequel le virus à ADN à double brin est un adénovirus.

20. Animal transgénique non humain ayant un ADN selon l'une quelconque des revendications 6 à 9 sur le chromosome.

21. Produit pharmaceutique comprenant l'ADN selon l'une quelconque des revendications 6 à 9.
